# EUROPÄISCHE PATENTANMELDUNG

(11) **EP 0 837 065 A1**
(43) Veröffentlichungstag der Anmeldung: **22.04.1998**
(21) Anmeldenummer: 97810746.4
(22) Anmeldetag: 07.10.1997
(51) Int. Cl.: C07D 405/12, C08K 5/3435

(54) **Phenylglycidylether-HALS**

(30) Priorität: 16.10.1996 CH 2524/96
(71) Anmelder: Ciba Specialty Chemicals Holding Inc., 4057 Basel (CH)
(72) Erfinder: Steinmann, Alfred, 1724 Praroman (CH)

(57) **Zusammenfassung**

Beschrieben werden Verbindungen der Formel I 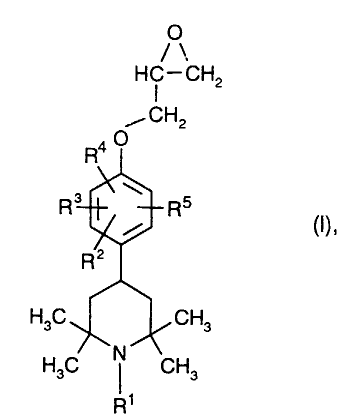 wobei
R¹ H, C₁-C₁₂-Alkyl, C₂-C₁₂-Alkenyl, C₂-C₁₂-Alkinyl, O-C₁-C₁₂-Alkyl, CO-C₁-C₁₂-Alkyl, wobei in den vorgenannten Resten Alkylketten auch durch O, S, S=O, SO₂ oder N-C₁-C₁₂-Alkyl unterbrochen sein können, C₅-C₁₂-Cycloalkyl, welches unsubstituiert oder durch 1 bis 4 C₁-C₄-Alkyl- und/oder C₁-C₄-Alkoxyreste substituiert ist, O-C₅-C₁₂-Cycloalkyl, welches unsubstituiert oder durch 1 bis 4 C₁-C₄-Alkyl- und/oder C₁-C₄-Alkoxyreste substituiert ist, CO-C₆-C₁₄-Aryl, das unsubstituiert oder durch 1 bis 9 C₁-C₄-Alkyl- und/oder C₁-C₄-Alkoxyreste substituiert ist, O-Benzyl, das am aromatischen Kern unsubstituiert oder durch 1 bis 4 C₁-C₄-Alkyl- und/oder C₁-C₄-Alkoxyreste substituiert ist, CO-Benzyl, das am aromatischen Kern unsubstituiert oder durch 1 bis 4 C₁-C₄-Alkyl- und/oder C₁-C₄-Alkoxyreste substituiert ist, und R², R³, R⁴ und R⁵ unabhängig voneinander H, C₁-C₁₂-Alkyl, C₅-C₁₂-Cycloalkyl, O-C₁-C₁₂-Alkyl, O-Phenyl, Halogen oder NO₂ bedeuten, sowie deren Homopolymere, Copolymere und Terpoylmere als auch die Umsetzungsprodukte mit mindestens einer weiteren nicht der Formel I entsprechenden Mono-Glycidylverbindung oder einem Amin oder einer Carbonsäure oder einem Phenol oder Dicarbonsäureanhydrid oder Alkoholat als auch Verbindungen, bei denen eine oder mehrere Verbindungen der Formel I an einen Füllstoff wie TiO₂, SiO₂, CaCO₃, CaSO₄, BaSO₄, Al(OH)₃, Talk, Russ, Glasfasern oder -kugeln, Cellulose oder Holzmehl adsorbiert ist/sind.

## Beschreibung

Gegenstand der Erfindung sind neue 4-[4-(2,3-Epoxypropoxy)phenyl]-2,2,6,6tetramethylpiperidine, die gegebenfalls am Ring-Stickstoff als auch am Phenylenring substituiert sind, deren Homopolymere, Copolymere und Terpolymere, deren Verwendung zum Stabilisieren von organischem Material gegen Schädigung durch Licht, Sauerstoff und/oder Wärme, sowie mit den genannten Verbindungen bzw. Polymeren stabilisiertes organisches Material.

Die Herstellung einiger Verbindungen vom Typ 4-(2,3-Epoxy-propoxy)-2,2,6,6-tetramethylpiperidin [4-Epoxy-HALS] sowie ihre Verwendung als Stabilisatoren für organische Polymere wird beispielsweise durch Luston und Vass, Makromolekulare Chemie, Macromol. Symp. 27, 231 (1989), beschrieben.

Reaktionsprodukte dieser Epoxyde mit Toluolsulfonsäure und Ethylendisulfonsäure, sowie deren kombinierte Verwendung als Härtungskatalysatoren und Lichtschutzmittel in Lacken sind in der EP-A-0 097 616 genannt.

Die Bindung reaktiver Alkylpiperidine an Fluorpolymere, die freie Carboxygruppen enthalten, wird in EP-A-526 399 beschrieben.

Die Publikation EP-A-001 835 lehrt die weitere Umsetzung der epoxydgruppenhaltigen Piperidine mit Dicarbonsäureanhydriden zu Polyestern.

Das US-Patent 5,521,282 beschreibt die anionische Polymerisation von 4-(2,3-Epoxy-propoxy)-2,2,6,6-tetramethylpiperidinen zu Polyethern mit gehinderten Amin-Seitenketten und deren Verwendung als Stabilisatoren. Die Poly(Glycidylether-HALS) haben überwiegend eine Erweichungstemperatur, die unter 20°C liegt, d. h. sie fallen bei ihrer Darstellung grösstenteils als Harze an.

Es besteht also weiterhin Bedarf an neuen, bei Raumtemperatur festen, Stabilisatoren vom HALS-Typ mit verbesserten Gebrauchseigenschaften, insbesondere auch besserer thermischer Beständigkeit als die der in US-A-5,521,282 genannten Verbindungen.

Es wurde nun gefunden, dass bestimmte Verbindungen vom Typ 4-[4-(2,3-Epoxypropoxy)phenyl]-2,2,6,6-tetramethylpiperidine sich überraschend gut als Stabilsatoren für organisches Material eignen. Sie weisen auch eine hohe Temperaturbeständigkeit auf und erlauben somit in der Anwendung höhere Verarbeitungstemperaturen und damit einen höheren Durchsatz bei der weiteren Verarbeitung der so stabilisierten Produkte.

Gegenstand der Erfindung sind daher zunächst Verbindungen der Formel I 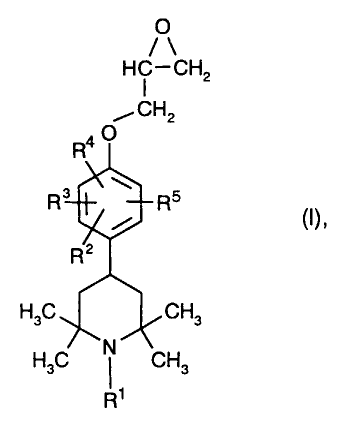 wobei
R¹ H, C₁-C₁₂-Alkyl, C₂-C₁₂-Alkenyl, C₂-C₁₂-Alkinyl, O-C₁-C₁₂-Alkyl, CO-C₁-C₁₂-Alkyl, wobei in den vorgenannten Resten Alkylketten auch durch O, S, S=O, SO₂ oder N-C₁-C₁₂-Alkyl unterbrochen sein können, C₅-C₁₂-Cycloalkyl, welches unsubstituiert oder durch 1 bis 4 C₁-C₄-Alkyl- und/oder C₁-C₄-Alkoxyreste substituiert ist, O-C₅-C₁₂-Cycloalkyl, welches unsubstituiert oder durch 1 bis 4 C₁-C₄-Alkyl- und/oder C₁-C₄-Alkoxyreste substituiert ist,
   CO-C₆-C₁₄-Aryl, das unsubstituiert oder durch 1 bis 9 C₁-C₄-Alkyl- und/oder C₁-C₄-Alkoxyreste substituiert ist, O-Benzyl, das am aromatischen Kern unsubstituiert oder durch 1 bis 4 C₁-C₄-Alkyl- und/oder C₁-C₄-Alkoxyreste substituiert ist, CO-Benzyl, das am aromatischen Kern unsubstituiert oder durch 1 bis 4 C₁-C₄-Alkyl- und/oder C₁-C₄-Alkoxyreste substituiert ist, und
R², R³, R⁴ und R⁵ unabhängig voneinander H, C₁-C₁₂-Alkyl, C₅-C₁₂-Cycloalkyl, O-C₁-C₁₂-Alkyl, O-Phenyl, Halogen oder NO₂ bedeuten.

R¹, R², R³, R⁴ und R⁵ als C₁-C₁₂-Alkyl können geradkettig oder verzweigt sein und bedeuten z. B. Methyl, Ethyl, Propyl, Isopropyl, n-Butyl, sec-Butyl, Isobutyl, t-Butyl, 2-Ethylbutyl, n-Pentyl, Isopentyl, 1-Methylpentyl, 1,3-Dimethylbutyl, n-Hexyl, 1-Methylhexyl, n-Heptyl, Isoheptyl, 1,1,3,3-Tetramethylbutyl, 1-Methylheptyl, 3-Methylheptyl, n-Octyl, 2-Ethylhexyl, 1,1,3-Trimethylhexyl, 1,1,3,3-Tetramethylpentyl, Nonyl, Decyl, Undecyl, 1-Methylundecyl, Dodecyl, 1,1,3,3,5,5-Hexamethylhexyl.
Die Alkylgruppe R¹ weist insbesondere 1 bis 8, vorzugsweise 1 bis 4 C-Atome auf. Die Alkylgruppen R², R³, R⁴ und R⁵ weisen insbesondere 1 bis 6 C-Atome auf.

Vorzugsweise bedeuten 2, insbesondere 3 der Substituenten R², R³, R⁴ und R⁵ Wasserstoff.

R¹ als C₂-C₁₂-Alkenyl bedeutet z. B. Vinyl, 1-Propenyl, 2-Propenyl, 1-Butenyl, 2-Butenyl, 3-Butenyl u.s.w. Bevorzugt sind Alkenylgruppen mit 2 bis 8 C-Atomen, insbesondere mit 2 bis 4 C-Atomen.

R¹ als C₂-C₁₂-Alkinyl bedeutet z. B. Ethinyl, 1-Propinyl, 2-Propinyl, 1-Butinyl, 2-Butinyl, 3-Butinyl u.s.w. Bevorzugt haben solche Alkinylgruppen 2 bis 8 C-Atome, besonders bevorzugt sind solche mit 2 bis 4 C-Atomen.

Die obengenannten Alkylketten können auch durch O, S, S=O, SO₂ oder N-C₁-C₁₂-Alkyl unterbrochen sein.

R¹ als C₅-C₁₂-Cycloalkyl bedeutet beispielsweise Cyclopentyl, Cyclohexyl, Cycloheptyl und Cyclooctyl u.s.w.
Bevorzugt ist Cyclohexyl.
Ist Cycloalkyl substituiert, trägt es vorzugsweise 2, insbesondere 1 Substituenten.
C₆-C₁₄-Aryl bedeutet z. B. Phenyl, Naphthyl, Anthracenyl, Phenanthrenyl.
Bevorzugt ist Phenyl.

R², R³, R⁴ und R⁵ als Halogen bedeutet F, Cl, Br und l, vorzugsweise Cl oder Br, vor allem aber Cl.

Bevorzugt sind Verbindung der Formel I, wobei
R¹ H, C₁-C₄-Alkyl, C₂-C₈-Alkenyl, C₂-C₄-Alkinyl, O-C₁-C₄-Alkyl, CO-C₁-C₄-Alkyl oder O-C₅-C₆-Cycloalkyl ist.

Besonders bevorzugt sind Verbindung der Formel I, bei denen
R¹ H, C₁-C₁₂-Alkyl, C₂-C₁₂-Alkenyl, C₂-C₁₂-Alkinyl, CO-C₁-C₁₂-Alkyl ist, wobei in den vorgenannten Resten Alkylketten auch durch O, S, S=O, SO₂ oder N-C₁-C₁₂-Alkyl unterbrochen sein können, und
R², R³, R⁴ und R⁵ unabhängig voneinander H, C₁-C₁₂-Alkyl oder O-C₁-C₁₂-Alkyl bedeuten.

Überaus bevorzugt sind Verbindung der Formel I, bei denen
R¹ H, CH₃, CH₂-C=CH oder CO-CH₃ und
R², R³, R⁴ und R⁵ unabhängig voneinander H oder CH3 bedeuten.

Ein weiterer Gegenstand der Erfindung sind Homopolymere, Copolymere und Terpolymere, erhältlich durch anionische Polymerisation von einer oder mehreren Verbindungen der Formel I.

Solche erfindungsgemässe Homopolymere, Copolymere und Terpolymere entsprechen vorzugsweise der Formel II 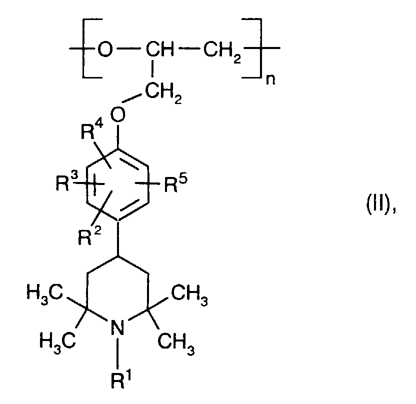 worin R¹, R², R³, R⁴ und R⁵ wie für Formel l definiert sind und n eine Zahl von 2 bis 1000 ist, und die allgemeinen Symbole in jeder wiederkehrenden Einheit gleich oder verschieden sind.

n ist in den Verbindungen der Formel II vorzugsweise 5 bis 500, insbesondere 5 bis 100.

Ein weiterer Gegenstand der Erfindung sind Verbindungen, erhältlich durch Umsetzung von Verbindungen der Formel I mit mindestens einer weiteren nicht der Formel l entsprechenden Glycidylverbindung, wie z. B. aliphatischen oder cycloaliphatischen Glycidylethern , beispielsweise n-Butylglycidylether oder Cyclohexylglycidylether, oder einer Glycidylether-Piperidinverbindung, wie beispielsweise im US-Patent 5,521,282 beschrieben.

Ein weiterer Gegenstand der Erfindung sind Verbindungen, erhältlich durch Umsetzung einer oder mehrerer Verbindungen der Formel I mit mindestens einem Amin oder einer Carbonsäure oder einem Phenol oder Dicarbonsäureanhydrid oder Alkoholat.

Beispiele dafür sind Verbindungen, erhältlich durch Umsetzung von Verbindungen der Formel I mit Piperidinen, Alkylaminen, Polyalkylenpolyaminen, Polyaminoamiden, Polyoxyalkylenpolyaminen, aromatischen Aminen oder nukleophile Aminogruppen enthaltenden Triazinen, Aminocarbonsäuren oder Ammoniak, wobei Piperidine, die mindestens zwei Stickstoffatome enthalten als auch Polyoxyalkylenpolyamine, die aminterminiertes Polyethylenglykol oder Polypropylenglykol oder beide Polyalkylenglykole beinhalten, aromatische Amine mit höchstens 2 aromatischen Kohlenstoffringen, an den C-Atomen mit Aminogruppen oder Aminogruppen enthaltenden Substituenten substituiertes Triazin, aromatische Aminocarbonsäuren oder Ammoniak bevorzugt sind.

Bevorzugt sind Piperidine der Formel III 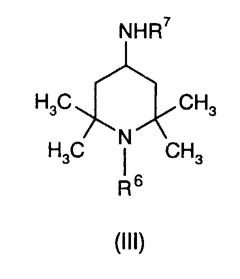 wobei
R⁶ H, C₁-C₁₂-Alkyl, C₂-C₁₂-Alkenyl, C₂-C₁₂-Alkinyl, C₆-C₂₀-Aryl, C₇C₂₀-Aralkyl, O-C₁-C₁₂-Alkyl,
O-C₅-C₈-Cycloalkyl, CO-C₁-C₁₂-Alkyl, CO-C₆-C₂₀-Aryl, CO-C₇C₂₀-Aralkyl, O-CO-C₁-C₁₂-Alkyl oder C₁-C₆-Alkyl-Z-C₁-C₆-Alkyl ist, wobei
Z O, S oder C=O ist und

R⁷ H, C₁-C₁₂-Alkyl oder 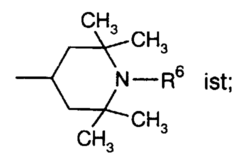 oder Alkylamine oder Dialkylamine, die maximal 20 C-Atome enthalten; Polyamine der Formeln IV bis VI 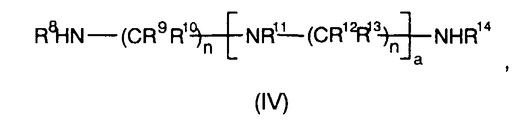 wobei
R⁸ bis R¹⁴ unabhängig voneinander H, CH₃, C₂H₅ oder C₃H₇ sind, und
R¹¹ zusätzlich R⁸HN-(CR⁹R¹⁰)n bedeuten kann,
n 2 bis 20 ist und
a 0 bis 30 ist, 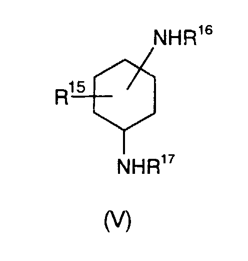
wobei
R¹⁵ bis R¹⁷ unabhängig voneinander H, CH₃ oder 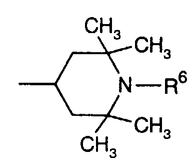 sind, oder 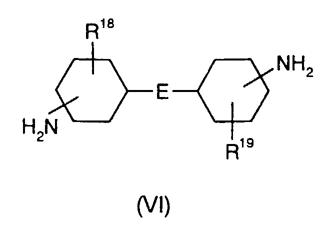
wobei
E eine direkte Bindung oder CH₂, C(CH₃)₂, C=O, N-H, S, SO, SO₂ oder O ist und
R¹⁸ und R¹⁹ unabhängig voneinander H, CH₃ oder 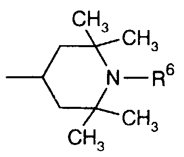 sind;
Polyaminoamide der Formel VII 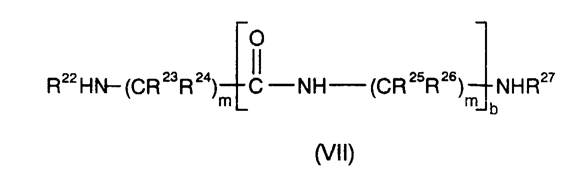
wobei
R²² bis R²⁷ unabhängig voneinander H oder CH₃ sind,
m 1 bis 20 ist und
b 1 bis 30 ist;

Polyoxyalkylenpolyamine der Formel VIII 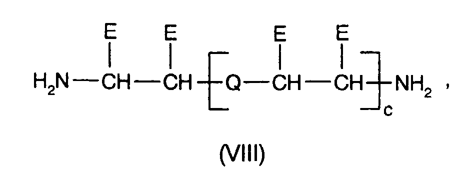 wobei
E unabhängig voneinander H oder CH3 ist und
Q unabhängig voneinander O oder NH ist und
c 1 bis 10.000 ist;
aromatische Amine der Formel IX 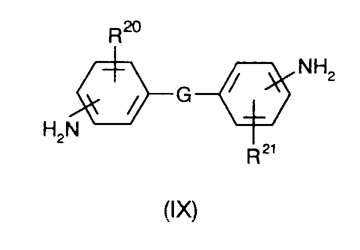
wobei
G eine direkte Bindung oder CH₂, C(CH₃)₂, C=O, N-H, S, SO, SO₂ oder O ist und
R²⁰ und R²¹ unabhängig voneinander H, CH₃ oder C₂H₅ sind;
substituierte Triazine der Formel X 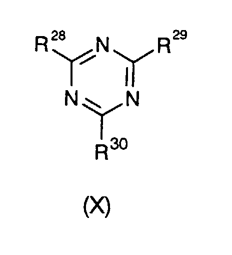
wobei
R²⁸ bis R³⁰ unabhängig voneinander einen primären und/oder sekundären Aminrest enthaltenden Substituenten mit einer C-Zahl von 1 bis 18 darstellen;
Aminocarbonsäuren der Formel XI 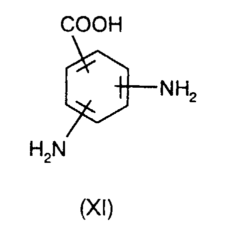 oder Ammoniak.

R⁶ und R⁷ der Formel III als C₁-C₁₂-Alkyl haben die gleiche Bedeutung wie R¹, R², R³, R⁴ und R⁵ als C₁-C₁₂-Alkyl der Verbindungen der Formeln I und II.

Alkylamine oder Dialkylamine, die maximal 20 C-Atome enthalten, bedeuten z. B. Methylamin, Ethylamin, n-Propylamin, Isopropylamin, n-Butylamin, Isobutylamin, tert.-Butylamin, n-Pentylamin, 2-Pentylamin, 3-Pentylamin, Pentaerythrylamin u.s.w. oder Dimethylamin, Diethylamin, Di-n-propylamin, Diisopropylamin, Di-n-butylamin, Diisobutylamin, Di-tert.-butylamin, Di-n-pentylamin, Di-2-pentylamin, Di-3-pentylamin, Di-pentaerythrylamin u.s.w. , d. h. Amine, deren C-Atomketten linear als auch verzweigt sein können. Es können auch Dialkylamine eingesetzt werden, die unterschiedliche Alkylketten, sowohl linear als auch verzweigt, enthalten, wie z. B. Methyl-isopropylamin.

Bei den Polyaminen der Formel IV handelt es sich um Polyamine wie beispielsweise Methylendiamin, 1,4-Butylendiamin, 2-Methylpentamethylendiamin, Hexamethylendiamin (HMD), Trimethylhexamethylendiamin (TMD), Bis-hexamethylentriamin (BHMD) oder Ethylendiamin (EDA), Diethylentriamin (DETA), Triethylentetramin (TETA), Tetraethylenpentamin (TEPA), Pentaethylenhexamin u.s.w. oder um 1,3-Propylendiamin, 1,2-Propylendiamin, Dipropylentriamin, Tripropylentetramin u.s.w. als auch um Amine, deren Alkylenketten verzweigt sind wie im Diisopropylentriamin. Die terminierenden Stickstoffatome können ebenso einen Alkylsubstituenten aus der Gruppe Methyl, Ethyl, n-Propyl oder Isopropyl tragen.
Ebenso sind Polyamine einsetzbar, welche cyclische Ringe beinhalten. Diese Ringe können sowohl aromatisch als auch cycloaliphatisch sein.

Typische einkernige aromatische Diamine sind beispielsweise die Xylylendiamine, wie z. B. m-Xylylendiamin (MXDA).

Typische Vertreter der cycloaliphatischen Diamine der Formel V sind das 1,2-Diaminocyclohexan (1,2-DACH) oder dessen Isomere bzw. das 1 -Methyl-2,4-diaminocyclohexan.

Isophorondiamin (IPD), 1,3-Bis-aminomethylcyclohexan (1,3-BAC) und 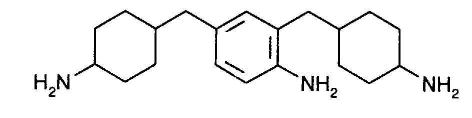 oder 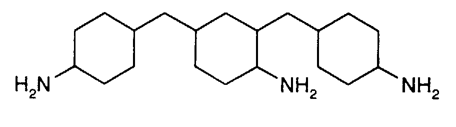 sind weitere einsetzbare cycloaliphatische Polyamine.

Beispiele für Polyamine der Formel VI sind:

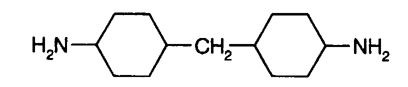

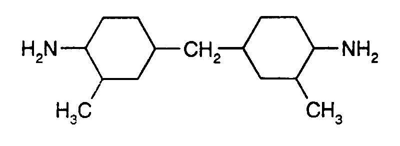

Norbornandiamin als auch N-2-Aminoethylpiperazin sind weitere typische Vertreter für Polyamine.

Polyaminoamide der Formel VII enthalten bis zu 30 wiederkehrende Amido-Einheiten, wobei die Kohlenstoffkette verzweigt sein kann.
Polyaminoamide können ebenso das Umsetzungsprodukt aus einer gegebenenfalls ungesättigten Fettsäure und einem Polyamin sein oder z. B. das Produkt aus Adipinsäure und Diethylentriamin, d. h. das Umsetzungsprodukt aus einer Dicarbonsäure und einem Polyamin.

Zu den Polyoxyalkylenpolyamine der Formel VIII, die für die Zwecke der vorliegenden Erfindung geeignet sind, gehören z. B. die Jeffamine® der Texaco Chemical Corporation [Texaco-Broschüre, Publikations-Nr. SC-024, 102-0548].

Beispiele für aromatische Amine der Formel IX sind 4,4'-Diaminodiphenylmethan (4,4'-DDM), 4,4'-Diaminodiphenylsulfon (4,4'-DDS), 4,4'-Diaminodiphenylether, 3,3'-Dimethyl-4,4'-diaminodiphenylmethan, 3,3'-Diethyl-4,4'-diaminodiphenylmethan u.s.w.

Aminocarbonsäuren können sowohl aliphatischer, cycloaliphatischer als auch aromatischer Natur sein; es können ebenso alle drei Strukturtypen in einem Molekül auftreten. Die aliphatischen Strukturen können sowohl geradkettig als auch verzweigt sein. Heteroatome können ebenso eingebaut sein.
Ein Beispiel für eine aromatische Aminocarbonsäure ist z. B. die 3,5-Diaminobenzoesäure.

Ein weiterer Gegenstand der Erfindung sind Verbindungen erhältlich durch Umsetzung von Verbindungen der Formel I mit einer Carbonsäure.

Es kann sich dabei um gegebenenfalls ungesättigte aliphatische als auch cycloaliphatische, aromatische oder gemischt aliphatisch-aromatische Säuren handeln, wobei die Zyklenzahl auch grösser zwei sein kann. Ebenfalls kann es sich bei den Carbonsäuren um mehrbasische Säuren handeln.

Es kann sich beispielsweise um folgende Säuren handeln:
Ameisensäure, Essigsäure, Hydroxyessigsäure, Aminoessigsäure, Chloressigsäure,
Trichloressigsäure, Thiolessigsäure, Propionsäure, 2,2-Dimethylpropansäure,
3-Chlorpropionsäure, Thiomilchsäure, 3-Thiolpropionsäure
3-Chlor-2,2-dimethylpropansäure, Buttersäure, Isobutansäure, 2-Methylbutansäure,
2-Ethylbutansäure, Valeriansäure, 2-Methylpentansäure, 2,2-Dimethylpentansäure,
2-Ethylhexansäure, 4-Oxopentansäure, 6-Aminohexansäure, 2-Bromhexansäure,
Isooctansäure, Isononansäure, Isodecansäure, Undecansäure, Stearinsäure,
1 2-Hydroxystearinsäure, 9-Decensäure, Ölsäure, Undec-1 0-encarbonsäure,
12-Hydroxyoctadec-9-ensäure, 2-Oxocyclopentancarbonsäure, Cyclohexancarbonsäure,
Cyclohexantricarbonsäure, Cyclohexylessigsäure,
all-cis-1,2,3,4-Cyclopentantetracarbonsäure, 2-Cyclopentenylessigsäure, Trimesinsäure,
Benzoesäure, o-Hydroxybenzoesäure, p-Hydroxybenzoesäure, o-Aminobenzoesäure,
p-Aminobenzoesäure, o-Nitrobenzoesäure, m-Nitrobenzoesäure, o-Chlorbenzoesäure,
p-Chlorbenzoesäure, 2,4-Dichlorbenzoesäure, Acetylsalicylsäure, Phenylessigsäure,
2-Hydroxyphenylessigsäure, Oxalsäure, Methylbernsteinsäure, Hydroxybernsteinsäure,
Chlorbernsteinsäure, Brombernsteinsäure, meso-2,3-Dibrombernsteinsäure,
Mercaptobernsteinsäure, 2-Oxoglutarsäure, Azelainsäure, Decandicarbonsäure,
Citronensäure, Methacrylsäure, Dimethylacrylsäure, Fumarsäure, Maleinsäure,
Methylmaleinsäure, Weinsäure, Phthalsäure, Isophthalsäure,
Hexachlornorbornendicarbonsäure, u.s.w.
Bevorzugt sind Citronensäure, Undecansäure, Cyclohexantricarbonsäure und Trimesinsäure.

Ein weiterer Gegenstand der Erfindung sind Verbindungen erhältlich durch Umsetzung von Verbindungen der Formel I mit einem Dicarbonsäureanhydrid.

Es kann sich dabei um gegebenenfalls ungesättigte aliphatische als auch cycloaliphatische, aromatische oder gemischt aliphatisch-aromatische Anhydride handeln, wobei die Zyklenzahl auch grösser zwei sein kann.

Beispielsweise kann es sich um folgende Dicarbonsäureanhydride handeln:
Essigsäureanhydrid, Propionsäureanhydrid, Maleinsäureanhydrid,
Methylmaleinsäureanhydrid, Dodec-2-enyl-maleinsäureanhydrid
2-Nonen-1-yl-bernsteinsäureanhydrid, 2-Dodecen-1-yl-bernsteinsäureanhydrid O,O'-Diacetylweinsäureanhydrid, 1 ,2-Cyclohexandicarbonsäureanhydrid,
4-Methyl-cis-1,2-cyclohexandicarbonsäureanhydrid, Tetrahydrophthalsäureanhydrid,
Phthalsäureanhydrid, 4-Chlorphthalsäureanhydrid,
4,5-Dichlorphthalsäureanhydrid, Tetrachlorphthalsäureanhydrid, Trimellitsäureanhydrid,
Norbornendicarbonsäureanhydrid, Hexachlornorbornendicarbonsäureanhydrid,
all-cis-1,2:3,4-Cyclopentantetracarbonsäureanhydrid, 1,2:4,5-Benzoltetracarbonsäuredianhydrid, u.s.w.
Bevorzugt sind 1,2-Cyclohexandicarbonsäureanhydrid, Phthalsäureanhydrid und Trimellitsäureanhydrid.

Ein weiterer Gegenstand der Erfindung sind Verbindungen erhältlich durch Umsetzung von Verbindungen der Formel I mit einem Phenol.

Beispielsweise kann es sich dabei um folgende Phenole handeln:
o-tert.-Butylphenol, p-Nonylphenol, p-Dodecenylphenol, o-Phenylphenol, p-Phenylphenol,
4-Chlor-2-methylphenol, 4-Chlor-3-metylphenol, o-Aminophenol, p-Aminophenol,
o-Nitrophenol, p-Nitrophenol, 2,4,6-Trinitrophenol, o-Chlorphenol, p-Chlorphenol,
2,4,6-Trichlorphenol, Tetrachlorphenol, Pentachlorphenol, 1-Naphthol, 2-Naphthol,
o-Hydroxybenzaldehyd, Brenzcatechin, Resorcin, Hydrochinon, Bisphenol-A, u.s.w.
Bevorzugt sind Resorcin und Bisphenol-A.

Ein weiterer Gegenstand der Erfindung sind Verbindungen erhältlich durch Umsetzung von Verbindungen der Formel I mit einem Metallalkoholat.
Bevorzugte Metallalkoholate sind beispielsweise Natriummethanolat, Natriumethanolat, Kaliummethanolat, Kaliumethanolat, Natriumisopropanolat oder Kalium-tert.-butanolat. Besonders bevorzugt sind Natriumethanolat oder Kalium-tert.-butanolat.

Für alle genannten Verbindungen gilt, dass alle möglichen Isomeren eingesetzt werden können.

Ein weiterer Gegenstand der Erfindung sind Verbindungen der Formel I, die an einen Füllstoff adsorbiert sind.
Der Füllstoff kann dabei beispielsweise TiO₂, SiO₂, CaCO₃, CaSO₄, BaSO₄, Al(OH)₃, Talk, Russ, Glasfasern oder -kugeln, Cellulose oder Holzmehl sein.

Die Herstellung der Epoxide der Formel I kann gemäss einer der Methoden erfolgen, die in der EP-A-0 634 399, EP-A-0 001 835 oder bei Luston und Vass, Makromolekulare Chemie, Macromol. Symp. 27, 231 (1989), beschrieben sind.

Zweckmässig wird eine Piperidinverbindung der Formel Y 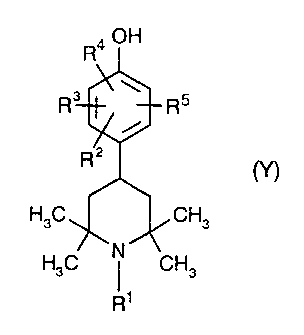 in Gegenwart starker Basen, beispielsweise wässriger konzentrierter Alkalihydroxidlösung, und gegebenenfalls eines organischen Lösungsmittels langsam mit einem Überschuss Epichlorhydrin versetzt.

Vorteilhaft wird die Base in ca. 2 bis 20 fachem molarem Überschuss bezogen auf die Ausgangsverbindung eingesetzt; beispielsweise werden 3 bis 15 Mol, bevorzugt 4 bis 12 Mol Natrium- oder Kaliumhydroxid als 50 % wässrige Lösung pro Mol Piperidinverbindung verwendet. Die Menge des organischen Lösemittels wird zweckmässig so bemessen, dass die Epoxidverbindung vollständig gelöst wird.

Die Reaktion erfolgt zweckmäßig in einem inerten Lösungsmittel. Als Lösungsmittel können polare oder unpolare organische Lösungsmittel eingesetzt werden wie z. B.
Kohlenwasserstoffe, Ether, Ketone, Amide, Nitrile, tert. Amine, halogenierte Kohlenwasserstoffe oder Sulfoxide; geeignet sind beispielsweise Toluol, Hexan, Cyclohexan, Ligroin, Petrolether und andere Kohlenwasserstoffgemische, Dimethylformamid, Methylenchlorid, Tetrahydrofuran, Dioxan, Diethylether, Dimethylsulfoxid und Acetonitril; besonders bevorzugt ist Methylenchlorid und Toluol.

Auf ein Äquivalent der Piperidinausgangsverbindung können beispielsweise 1 bis 4, vorzugsweise 1,2 bis 3, vor allem 1,5 bis 2,5 Äquivalente Epichlorhydrin eingesetzt werden. Darüber hinaus können der Mischung vorteilhaft 0,01 bis 10 Mol-%, bevorzugt 2 bis 4 Mol-%, eines tert. Aminsalzes, beispielsweise eines Tetraalkylammoniumhalogenids wie Tetramethylammoniumchlorid oder Tetrabutylammoniumbromid, oder eines Phosphoniumsalzes, beispielsweise eines quartären Phosphoniumhalogenids wie Ethyltriphenylphosphoniumbromid, als Katalysator zugesetzt werden.

Die Temperatur kann während der Reaktion im Bereich von 0 bis 120°C liegen, zweckmässig beträgt die Temperatur 10 bis 80°C, vor allem 20 bis 70°C.

Zur Herstellung der Ausgangsverbindungen der Formel Y geht man beispielsweise von kommerziell erhältlichem Triacetonamin aus und setzt dieses mit Phenol in konzentrierter salzsaurer Lösung zweckmässig bei erhöhter Temperatur um (C. A. 90:54839g). Die Reduktion des Hydrochlorids mit Wasserstoff mittels eines Katalysators ergibt dann das gegebenenfalls substituierte 4-(4-Hydroxyphenyl)-2,2,6,6-tetramethylpiperidin (DE-A-2 258 86). Zur Einführung eines Substituenten am Piperidinstickstoff muss die Hydroxygruppe des Phenylringes in an sich bekannter Weise, beispielsweise mit Hilfe einer Timethylsilylgruppe geschützt werden.

Die Herstellung der Homopolymeren, Copolymeren und Terpolymeren von Verbindungen der Formel I erfolgt zweckmässig unter anionischer Polymerisation, wobei insbesondere Polyether der Formel II entstehen. Diese können vollständig aus gleichen wiederkehrenden Einheiten linear aufgebaut sein oder auch modifiziert sein.

Die Polymerisation kann beispielsweise nach einer der von K. C. Frisch und S. L. Reegen beschriebenen Methoden ausgeführt werden (Frisch/Reegen: RingOpening Polymerization, Marcel Dekker, New York 1969). Die Polymerisation wird in der Regel durch einen der üblichen Initiatoren der anionischen Polymerisation eingeleitet. Dazu zählen basische metallorganische Verbindungen wie GrignardVerbindungen, beispielsweise vom Typ Cl-Mg-C₁-C₁₂-Alkyl oder Cl-Mg-C₆-C₁₂-Aryl, Alkalialkyle, beispielsweise C₁-C₆-Alkalialkyle wie Kalium-tert.-butylat, Alkalialkoholate Me-OR', worin Me beispielsweise Li, Na, K, und R' C₁-C₆-Alkyl ist, wie z. B. Natrium- oder Kaliummethanolat, Natrium- oder Kalium-tert.-butylat, Lithium- oder Natriumethanolat, Hydroxide oder Amide wie beispielsweise NaOH, KOH, Natriumamid, Lithiumamid.

Der Initiator wird zweckmässig in einer Menge von 0,1 - 20 Mol-%, vorzugsweise 1 - 10 Mol-%, zugesetzt, bezogen auf die Menge an Epoxid der Formel I.

Der Mischung kann ein Kronenether wie 18-Krone-6 (18-Crown-6) oder 15-Krone-5 (15-Crown-5) zugesetzt werden, zweckmässig in einer Menge von 0,1 - 10 Mol-%, vorzugsweise 1 - 5 Mol-%, bezogen auf die Menge an Epoxid der Formel I.

Die Molekulargewichte können durch die Menge zugesetzten Initiators kontrolliert werden.

Die Polymerisation kann ohne Lösemittel durchgeführt werden; die Verwendung eines Lösemittels ist jedoch bevorzugt. Die Reaktionstemperatur ist nicht kritisch; sie bewegt sich im allgemeinen im Bereich von 10 bis 200°C.

Gegebenenfalls anwesende Lösemittel müssen unter den Reaktionsbedingungen inert sein. Als Lösemittel kommen u. a. aromatische und/oder aliphatische Kohlenwasserstoffe und Ether in Betracht. Bevorzugt sind hochsiedende Lösemittel, beispielsweise solche, deren Siedetemperatur bei Normaldruck im Bereich 80 - 150°C liegt. Beispiele für verwendbare Lösemittel sind Benzol, Toluol, Xylol, Ethylbenzol, Isopropylbenzol, Cyclohexan, Diethylether, Dibutylether, Tetrahydrofuran oder Dioxan.

Die Polymerisation wird zweckmässig unter Ausschluß von Sauerstoff durchgeführt, beispielsweise unter Argon oder unter Stickstoff, sowie unter Ausschluss von Wasser.

Nach vollständiger Polymerisation kann die Aufarbeitung nach gängigen Methoden erfolgen; zweckmässig wird die Mischung zunächst mit einem geeigneten Lösemittel, beispielsweise Tetrahydrofuran, verdünnt. Die Lösung kann durch Filtration, gegebenenfalls nach Dispergieren von Aktivkohle, gereinigt werden. Aus der Lösung lässt sich das Polymer mit Hilfe eines weiteren Lösemittels geeigneter Polarität, beispielsweise Acetonitril, ausfällen; dies kann durch Eintragen der Polymerlösung in eine grössere Menge des Fällungsmittels geschehen. Die Reinigung durch Fällung kann bei Bedarf mehrfach wiederholt werden.
Von den gewählten Polymerisations- und Aufarbeitungsbedingungen hängt ab, welche Endgruppen die erfindungsgemässen Polyether der Formel II aufweisen. Die terminalen Kohlenstoffatome der Polyetherkette können beispielsweise durch -H oder -OH oder einen Rest der als Initiator verwendeten Verbindung abgesättigt sein. Wird als Initiator beispielsweise eines der oben beschriebenen Alkoholate R'O- eingesetzt und im Anschluss an die Polymerisation mit einem protischen Lösemittel aufgearbeitet, können häufig die Endgruppen -OR' und -OH an den terminalen Kohlenstoffatomen auftreten.

Prinzipiell ist die Art der Endgruppe für die Wirkung der erfindungsgemässen Polyether als Stabilisator jedoch von untergeordneter Bedeutung.

Die Herstellung der erfindungsgemässen Amino-Addukte erfolgt in einer Reaktion zweckmässig ohne Lösungsmittel und bei erhöhter Temperatur.
Die Temperatur kann während der Reaktion im Bereich von 80 bis 250°C liegen, zweckmässig beträgt die Temperatur 100 bis 200°C, vor allem 170 bis 190°C. Bei Verbindungen mit R¹ = O-C₁-C₁₂-Alkyl, O-C₅-C₈-Cycloalkyl oder O-CO-C₁-C₁₂-Alkyl liegt die Reaktionstemperatur unter 100°C.

Bei der Reaktion der Verbindungen der Formel I mit primären oder sekundären Aminen, die mehr als einen reaktionsfähigen Stickstoff enthalten, können alle oder auch nur einige oder eine der Aminofunktionen mit dem eine Epoxidfunktion enthaltenden Piperidin abreagieren. Ebenso können für die Reaktion Mischungen von zwei oder mehr Aminen eingesetzt werden, die im obigen Sinne abreagieren können.

Die Umsetzung der erfindungsgemässen Verbindungen der Formel I mit Carbonsäuren, Dicarbonsäureanhydriden als auch Phenolen erfolgt zweckmässigerweise analog wie im EP-A-0 634 399 beschrieben.

Die Umsetzung der erfindungsgemässen Verbindungen der Formel I mit Alkalimetallalkoholaten erfolgt zweckmässigerweise mit Natriumethanolat oder Kalium-tert.-butanolat. Je nach Molverhältnis zwischen Alkoholat und Glycidylether können die entstehenden Produkte 1:1-Addukte bis zu Polymeren sein.

Die Adsorption der erfindungsgemässen Verbindungen der Formel I an mindestens einen Füllstoff erfolgt zweckmässigerweise in Lösung durch Impregnation des Füllstoffes und anschliessendem Abdampfen des Lösungsmittels.

Falls die Reaktionen in einem inerten Lösungsmittel ausgeführt werden, so kann die Temperatur der Reaktionsmischung für die Dauer der Reaktion im Siedebereich gehalten werden (Rückfluß). Dazu wird eine Lösemittel enthaltende Reaktionsmischung, im allgemeinen unter Normaldruck, zum Siedepunkt erwärmt und das verdampfte Lösemittel mit Hilfe eines geeigneten Kühlers kondensiert und wieder der Reaktionsmischung zugeführt.

Die Reaktionen können unter Ausschluß von Sauerstoff durchgeführt werden, beispielsweise durch Spülen mit einem Inertgas wie Argon; Sauerstoff stört jedoch nicht in jedem Fall, so daß die Reaktion auch ohne die genannte Maßnahme durchgeführt werden kann.

Nach beendeter Reaktion kann die Aufarbeitung nach gängigen Methoden erfolgen; zweckmässig wird die Mischung zunächst mit Wasser verdünnt, beispielsweise indem das Reaktionsgemisch in das 1-4-fache Volumen (Eis-)Wasser gegeben wird; anschliessend kann das Produkt direkt abgetrennt oder extrahiert werden, zur Extraktion eignet sich z. B. Essigester oder Toluol. Wird extrahiert, so kann das Produkt in üblicher Weise durch Entfernen des Lösemittels isoliert werden; dies geschieht zweckmässig nach Trocknen der organischen Phase. Möglich ist auch das Einschalten weiterer Reinigungsschritte wie beispielsweise Waschen mit wässriger Natriumbicarbonatlösung, Dispergieren von Aktivkohle, chromatographieren mittels Kieselgels, Filtrieren, Umkristallisieren und/oder Destillieren.

Bei der Reaktion der Verbindungen der Formel I mit primären oder sekundären Aminen oder Ammoniak oder einer Carbonsäure, einem Dicarbonsäureanhydrid, einem Phenol oder Alkalimetallalkoholat entstehen hauptsächlich Verbindungen, die folgende Strukturelemente A und/oder B enthalten: 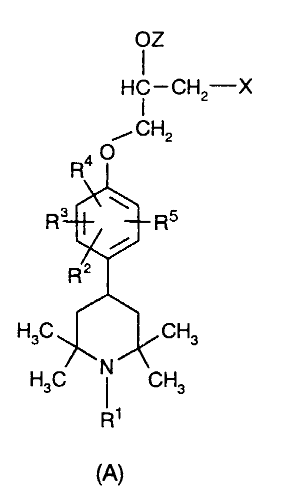 oder 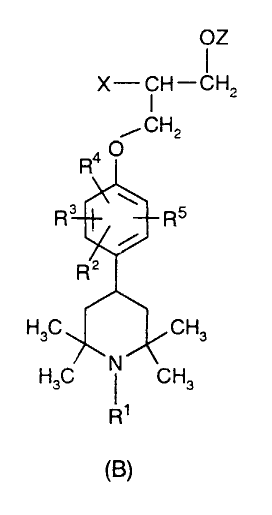 wobei Z Wasserstoff oder ein Alkalimetall und X das Symbol für den nach der Abtrennung von Z verbleibenden nucleophilen Rest des Amins oder der Carbonsäure oder des Phenols oder Dicarbonsäureanhydrids oder Alkoholats darstellen und R¹, R², R³, R⁴ und R⁵ die unter Formel I gegebene Definition haben. Die Verbindungen der Formel A werden dabei bevorzugt gebildet und stellen somit das Hauptprodukt dar.

Die Verbindungen der Formel I sowie deren erfindungsgemässen Homo-, Co- und Terpolymeren sowie die Verbindungen, erhältlich durch Umsetzung einer oder mehrerer Verbindungen der Formel I mit mindestens einer weiteren nicht der Formel I entsprechenden Mono-Glycidylverbindung als auch Verbindungen, erhältlich durch Umsetzung einer oder mehrerer Verbindungen der Formel I mit mindestens einem Amin oder einer Carbonsäure oder einem Phenol oder Dicarbonsäureanhydrid oder Alkoholat sowie Verbindungen der Formel I, die an einen Füllstoff adsorbiert sind, eignen sich besonders zum Stabilisieren von organischen Materialien gegen thermischen, oxidativen und aktinischen Abbau.

Beispiele für derartige Materialien sind:
1. Polymere von Mono- und Diolefinen, beispielsweise Polypropylen, Polyisobutylen, Polybuten-1, Poly-4-methylpenten-1, Polyisopren oder Polybutadien sowie Polymerisate von Cycloolefinen wie z.B. von Cyclopenten oder Norbornen; ferner Polyethylen (das gegebenenfalls vernetzt sein kann), z.B. Polyethylen hoher Dichte (HDPE), Polyethylen niederer Dichte (LDPE), lineares Polyethylen niederer Dichte (LLDPE), verzweigtes Polyethylen niederer Dichte (VLDPE).
   Polyolefine, d.h. Polymere von Monoolefinen, wie sie beispielhaft im vorstehenden Absatz erwähnt sind, insbesondere Polyethylen und Polypropylen, können nach verschiedenen Verfahren hergestellt werden, insbesondere nach den folgenden Methoden:
   a) radikalisch (gewöhnlich bei hohem Druck und hoher Temperatur).
   b) mittels Katalysator, wobei der Katalysator gewöhnlich ein oder mehrere Metalle der Gruppe IVb, Vb, VIb oder VIII enthält. Diese Metalle besitzen gewöhnlich einen oder mehrere Liganden wie Oxide, Halogenide, Alkoholate, Ester, Ether, Amine, Alkyle, Alkenyle und/oder Aryle, die entweder π- oder σ-koordiniert sein können. Diese Metallkomplexe können frei oder auf Träger fixiert sein, wie beispielsweise auf aktiviertem Magnesiumchlorid, Titan(III)chlorid, Aluminiumoxid oder Siliziumoxid. Diese Katalysatoren können im Polymerisationsmedium löslich oder unlöslich sein. Die Katalysatoren können als solche in der Polymerisation aktiv sein, oder es können weitere Aktivatoren verwendet werden, wie beispielsweise Metallalkyle, Metallhydride, Metallalkylhalogenide, Metallalkyloxide oder Metallalkyloxane, wobei die Metalle Elemente der Gruppen Ia, IIa und/oder IIIa sind. Die Aktivatoren können beipielsweise mit weiteren Ester-, Ether-, Amin- oder Silylether-Gruppen modifiziert sein. Diese Katalysatorsysteme werden gewöhnlich als Phillips, Standard Oil Indiana, Ziegler(-Natta), TNZ (DuPont), Metallocen oder Single Site Katalysatoren (SSC) bezeichnet.
2. Mischungen der unter 1) genannten Polymeren, z.B. Mischungen von Polypropylen mit Polyisobutylen, Polypropylen mit Polyethylen (z.B. PP/HDPE, PP/LDPE) und Mischungen verschiedener Polyethylentypen (z.B. LDPE/HDPE).
3. Copolymere von Mono- und Diolefinen untereinander oder mit anderen Vinylmonomeren, wie z.B. Ethylen-Propylen-Copolymere, lineares Polyethylen niederer Dichte (LLDPE) und Mischungen desselben mit Polyethylen niederer Dichte (LDPE), Propylen--Buten-1-Copolymere, Propylen-lsobutylen-Copolymere, Ethylen-Buten-1-Copolymere, Ethylen-Hexen-Copolymere, Ethylen-Methylpenten-Copolymere, Ethylen-Hepten-Copolymere, Ethylen-Octen-Copolymere, Propylen-Butadien-Copolymere, Isobutylen-Isopren--Copolymere, Ethylen-Alkylacrylat-Copolymere, Ethylen-Alkylmethacrylat- Copolymere, Ethylen-Vinylacetat-Copolymere und deren Copolymere mit Kohlenstoffmonoxid, oder Ethylen-Acrylsäure-Copolymere und deren Salze (Ionomere), sowie Terpolymere von Ethylen mit Propylen und einem Dien, wie Hexadien, Dicyclopentadien oder Ethylidennorbornen; ferner Mischungen solcher Copolymere untereinander und mit unter 1) genannten Polymeren, z.B. Polypropylen/Ethylen-Propylen-Copolymere, LDPE/Ethylen-Vinylacetat-Copolymere, LDPE/Ethylen-Acrylsäure-Copolymere, LLDPE/Ethylen-Vinylacetat-Copolymere, LLDPE/Ethylen-Acrylsäure-Copolymere und alternierend oder statistisch aufgebaute Polyalkylen/Kohlenstoffmonoxid-Copolymere und deren Mischungen mit anderen Polymeren wie z.B. Polyamiden.
4. Kohlenwasserstoffharze (z. B. C₅-C₉) inklusive hydrierte Modifikationen davon (z.B. Klebrigmacherharze) und Mischungen von Polyalkylenen und Stärke.
5. Polystyrol, Poly-(p-methylstyrol), Poly-(a-methylstyrol).
6. Copolymere von Styrol oder α-Methylstyrol mit Dienen oder Acrylderivaten, wie z.B. Styrol-Butadien, Styrol-Acrylnitril, Styrol-Alkylmethacrylat, Styrol-Butadien-Alkylacrylat und - methacrylat, Styrol-Maleinsäureanhydrid, Styrol-Acrylnitril-Methylacrylat; Mischungen von hoher Schlagzähigkeit aus Styrol-Copolymeren und einem anderen Polymer, wie z.B. einem Polyacrylat, einem Dien-Polymeren oder einem Ethylen-Propylen-Dien-Terpolymeren; sowie
   Block-Copolymere des Styrols, wie z.B. Styrol-Butadien-Styrol, Styrol-Isopren-Styrol, Styrol-Ethylen/Butylen-Styrol oder Styrol-Ethylen/Propylen-Styrol.
7. Pfropfcopolymere von Styrol oder α-Methylstyrol, wie z.B. Styrol auf Polybutadien, Styrol auf Polybutadien-Styrol- oder Polybutadien-Acrylnitril-Copolymere, Styrol und Acrylnitril (bzw. Methacrylnitril) auf Polybutadien; Styrol, Acrylnitril und Methylmethacrylat auf Polybutadien; Styrol und Maleinsäureanhydrid auf Polybutadien; Styrol, Acrylnitril und Maleinsäureanhydrid oder Maleinsäureimid auf Polybutadien; Styrol und Maleinsäureimid auf Polybutadien, Styrol und Alkylacrylate bzw. Alkylmethacrylate auf Polybutadien, Styrol und Acrylnitril auf Ethylen-Propylen-Dien-Terpolymeren, Styrol und Acrylnitril auf Polyalkylacrylaten oder Polyalkylmethacrylaten, Styrol und Acrylnitril auf Acrylat-Butadien-Copolymeren, sowie deren Mischungen mit den unter 6) genannten Copolymeren, wie sie z.B. als sogenannte ABS-, MBS-, ASA- oder AES-Polymere bekannt sind.
8. Halogenhaltige Polymere, wie z.B. Polychloropren, Chlorkautschuk, chloriertes oder chlorsulfoniertes Polyethylen, Copolymere von Ethylen und chloriertem Ethylen, Epichlorhydrinhomo- und -copolymere, insbesondere Polymere aus halogenhaltigen Vinylverbindungen, wie z.B. Polyvinylchlorid, Polyvinylidenchlorid, Polyvinylfluorid, Polyvinylidenfluorid; sowie deren Copolymere, wie Vinylchlorid-Vinylidenchlorid, Vinylchlorid-Vinylacetat oder Vinylidenchlorid-Vinylacetat.
9. Polymere, die sich von α,β-ungesättigten Säuren und deren Derivaten ableiten, wie Polyacrylate und Polymethacrylate, mit Butylacrylat schlagzäh modifizierte Polymethylmethacrylate, Polyacrylamide und Polyacrylnitrile.
10. Copolymere der unter 9) genannten Monomeren untereinander oder mit anderen ungesättigten Monomeren, wie z.B. Acrylnitril-Butadien-Copolymere, Acrylnitril-Alkylacrylat-Copolymere, Acrylnitril-Alkoxyalkylacrylat-Copolymere, Acrylnitril-Vinylhalogenid-Copolymere oder Acrylnitril-Al kylmethacrylat-Butadien-Terpolymere.
11. Polymere, die sich von ungesättigten Alkoholen und Aminen bzw. deren Acylderivaten oder Acetalen ableiten, wie Polyvinylalkohol, Polyvinylacetat, -stearat, -benzoat, -maleat, Polyvinylbutyral, Polyallylphthalat, Polyallylmelamin; sowie deren Copolymere mit in Punkt 1 genannten Olefinen.
12. Homo- und Copolymere von cyclischen Ethern, wie Polyalkylenglykole, Polyethylenoxyd, Polypropylenoxyd oder deren Copolymere mit Bisglycidylethern.
13. Polyacetale, wie Polyoxymethylen, sowie solche Polyoxymethylene, die Comonomere, wie z.B. Ethylenoxid, enthalten; Polyacetale, die mit thermoplastischen Polyurethanen, Acrylaten oder MBS modifiziert sind.
14. Polyphenylenoxide und -sulfide und deren Mischungen mit Styrolpolymeren oder Polyamiden.
15. Polyurethane, die sich von Polyethern, Polyestern und Polybutadienen mit endständigen Hydroxylgruppen einerseits und aliphatischen oder aromatischen Polyisocyanaten andererseits ableiten, sowie deren Vorprodukte.
16. Polyamide und Copolyamide, die sich von Diaminen und Dicarbonsäuren und/oder von Aminocarbonsäuren oder den entsprechenden Lactamen ableiten, wie Polyamid 4, Polyamid 6, Polyamid 6/6, 6/10, 6/9, 6/12, 4/6, 12/12, Polyamid 11, Polyamid 12, aromatische Polyamide ausgehend von m-Xylol, Diamin und Adipinsäure; Polyamide, hergestellt aus Hexamethylendiamin und Iso- und/oder Terephthalsäure und gegebenenfalls einem Elastomer als Modifikator, z.B. Poly-2,4,4-trimethylhexamethylenterephthalamid oder Poly-mphenylen-isophthalamid. Block-Copolymere der vorstehend genanntenPolyamide mit Polyolefinen, Olefin-Copolymeren, Ionomeren oder chemisch gebundenen oder gepfropften Elastomeren; oder mit Polyethern, wie z.B. mit Polyethylenglykol, Polypropylenglykol oder Polytetramethylenglykol. Ferner mit EPDM oder ABS modifizierte Polyamide oder Copolyamide; sowie während der Verarbeitung kondensierte Polyamide ("RIM-Polyamidsysteme").
17. Polyharnstoffe, Polyimide, Polyamid-imide und Polybenzimidazole.
18. Polyester, die sich von Dicarbonsäuren und Dialkoholen und/oder von Hydroxycarbonsäuren oder den entsprechenden Lactonen ableiten, wie Polyethylenterephthalat, Polybutylenterephthalat, Poly-1,4-dimethylolcyclohexanterephthalat, Polyhydroxybenzoate, sowie Block-Polyether-ester, die sich von Polyethern mit Hydroxylendgruppen ableiten; ferner mit Polycarbonaten oder MBS modifizierte Polyester.
19. Polycarbonate und Polyestercarbonate.
20. Polysulfone, Polyethersulfone und Polyetherketone.
21. Vernetzte Polymere, die sich von Aldehyden einerseits und Phenolen, Harnstoff oder Melamin andererseits ableiten, wie Phenol-Formaldehyd-, Harnstoff-Formaldehyd- und Melamin-Formaldehydharze.
22. Trocknende und nicht-trocknende Alkydharze.
23. Ungesättigte Polyesterharze, die sich von Copolyestern gsättigter und ungesättigter Dicarbonsäuren mit mehrwertigen Alkoholen, sowie Vinylverbindungen als Vernetzungsmittel ableiten, wie auch deren halogenhaltige, schwerbrennbare Modifikationen.
24. Vernetzbare Acrylharze, die sich von substituierten Acrylsäureestern ableiten, wie z.B. von Epoxyacrylaten, Urethan-acrylaten oder Polyester-acrylaten.
25. Alkydharze, Polyesterharze und Acrylatharze, die mit Melaminharzen, Harnstoffharzen, Isocyanaten, Isocyanuraten, Polyisocyanaten oder Epoxidharzen vernetzt sind.
26. Vernetzte Epoxidharze, die sich von Polyepoxiden ableiten, z.B. von Bis-glycidylethern oder von cycloaliphatischen Diepoxiden.
27. Natürliche Polymere, wie Cellulose, Naturkautschuk, Gelatine, sowie deren polymerhomolog chemisch abgewandelte Derivate, wie Celluloseacetate, -propionate und -butyrate, bzw. die Celluloseether, wie Methylcellulose; sowie Kolophoniumharze und Derivate.
28. Mischungen (Polyblends) der vorgenannten Polymeren, wie z.B. PP/EPDM, Polyamid/EPDM oder ABS, PVC/EVA, PVC/ABS, PVC/MBS, PC/ABS, PBTP/ABS, PC/ASA, PC/PBT, PVC/CPE, PVC/Acrylate, POM/thermoplastisches PUR, PC/thermoplastisches PUR, POM/Acrylat, POM/MBS, PPO/HIPS, PPO/PA 6.6 und Copolymere, PA/HDPE, PA/PP, PA/PPO.

Weitere Gegenstände der Erfindung sind daher Zusammensetzungen enthaltend a) ein gegen oxidativen, thermischen oder/und aktinischen Abbau/Aufbau empfindliches organisches Material und b) mindestens eine Verbindung der Formel I sowie deren erfindungsgemässen Homo-, Co- und Terpolymeren sowie die Verbindungen, erhältlich durch Umsetzung einer oder mehrerer Verbindungen der Formel I mit mindestens einer weiteren nicht der Formel I entsprechenden Mono-Glycidylverbindung als auch Verbindungen, erhältlich durch Umsetzung einer oder mehrerer Verbindungen der Formel I mit mindestens einem Amin oder einer Carbonsäure oder einem Phenol oder Dicarbonsäureanhydrid oder Alkoholat sowie Verbindungen der Formel I, die an einen Füllstoff adsorbiert sind, sowie die Verwendung von Verbindungen der Formel I sowie deren erfindungsgemässen Homo-, Co- und Terpolymeren sowie die Verbindungen, erhältlich durch Umsetzung einer oder mehrerer Verbindungen der Formel I mit mindestens einer weiteren nicht der Formel I entsprechenden Mono-Glycidylverbindung als auch Verbindungen, erhältlich durch Umsetzung einer oder mehrerer Verbindungen der Formel I mit mindestens einem Amin oder einer Carbonsäure oder einem Phenol oder Dicarbonsäureanhydrid oder Alkoholat sowie Verbindungen der Formel I, die an einen Füllstoff adsorbiert sind, zum Stabilisieren von organischem Material gegen oxidativen, thermischen oder aktinischen Abbau/Aufbau.
Die Erfindung umfaßt ebenfalls ein Verfahren zum Stabilisieren von organischem Material gegen thermischen, oxidativen oder/und aktinischen Abbau/Aufbau, dadurch gekennzeichnet, daß man diesem Material mindestens eine Verbindung der Formel I sowie deren erfindungsgemässen Homo-, Co- und Terpolymeren sowie die Verbindungen, erhältlich durch Umsetzung einer oder mehrerer Verbindungen der Formel I mit mindestens einer weiteren nicht der Formel I entsprechenden Mono-Glycidylverbindung als auch Verbindungen, erhältlich durch Umsetzung einer oder mehrerer Verbindungen der Formel I mit mindestens einem Amin oder einer Carbonsäure oder einem Phenol oder Dicarbonsäureanhydrid oder Alkoholat sowie Verbindungen der Formel I, die an einen Füllstoff adsorbiert sind, zusetzt.

Von besonderem Interesse ist die Verwendung von Verbindungen der Formel I als Stabilisatoren in synthetischen organischen Polymeren sowie entsprechende Zusammensetzungen.

Vorzugsweise handelt es sich bei den zu schützenden organischen Materialien um natürliche, halbsynthetische oder bevorzugt synthetische organische Materialien. Besonders bevorzugt sind synthetische organische Polymere oder Gemische solcher Polymere, insbesondere thermoplastische Polymere wie Polyolefine, vor allem Polyethylen (PE) und Polypropylen (PP). Ebenfalls besonders bevorzugte organische Materialien sind Überzugszusammensetzungen. Im Sinne der Erfindung vorteilhaft zu stabilisierende Überzugszusammensetzungen sind beispielsweise beschrieben in Ullmann's Encyclopedia of Industrial Chemistry, 5. Ed., Vol. A18, pp. 359-464, VCH Verlagsgesellschaft, Weinheim 1991.

Von besonderem Interesse ist die Verwendung der erfindungsgemäßen Verbindungen der Formel I sowie deren erfindungsgemässen Homo-, Co- und Terpolymeren sowie die Verbindungen, erhältlich durch Umsetzung einer oder mehrerer Verbindungen der Formel I mit mindestens einer weiteren nicht der Formel I entsprechenden Mono-Glycidylverbindung als auch Verbindungen, erhältlich durch Umsetzung einer oder mehrerer Verbindungen der Formel I mit mindestens einem Amin oder einer Carbonsäure oder einem Phenol oder Dicarbonsäureanhydrid oder Alkoholat sowie Verbindungen der Formel I, die an einen Füllstoff adsorbiert sind, als Stabilisatoren für Überzüge, beispielsweise für Lacke. Gegenstand der Erfindung sind daher auch solche Zusammensetzungen, deren Komponente A ein filmbildendes Bindemittel ist.

Das erfindungsgemäße Überzugsmittel enthält vorzugsweise auf 100 Gew.-Teile festes Bindemittel A 0,01-10 Gew.-Teile, insbesondere 0,05-10 Gew.-Teile, vor allem 0,1-5 Gew.-Teile des erfindungsgemäßen Stabilisators B.

Auch hier sind Mehrschichtsysteme möglich, wobei die Konzentration der Verbindung der Formel I, sowie deren erfindungsgemässen Homo-, Co- und Terpolymeren sowie die Verbindungen, erhältlich durch Umsetzung einer oder mehrerer Verbindungen der Formel I mit mindestens einer weiteren nicht der Formel I entsprechenden Mono-Glycidylverbindung als auch Verbindungen, erhältlich durch Umsetzung einer oder mehrerer Verbindungen der Formel I mit mindestens einem Amin oder einer Carbonsäure oder einem Phenol oder Dicarbonsäureanhydrid oder Alkoholat sowie Verbindungen der Formel I, die an einen Füllstoff adsorbiert sind, (Komponente B) in der Deckschicht höher sein kann, beispielsweise 1 bis 15 Gew.-Teile, vor allem 3-10 Gew.-Teile B auf 100 Gew.-Teile festes Bindemittel A.

Die Verwendung der Verbindung der Formel I, sowie deren erfindungsgemässen Homo-, Co- und Terpolymeren sowie die Verbindungen, erhältlich durch Umsetzung einer oder mehrerer Verbindungen der Formel I mit mindestens einer weiteren nicht der Formel I entsprechenden Mono-Glycidylverbindung als auch Verbindungen, erhältlich durch Umsetzung einer oder mehrerer Verbindungen der Formel 1 mit mindestens einem Amin oder einer Carbonsäure oder einem Phenol oder Dicarbonsäureanhydrid oder Alkoholat sowie Verbindungen der Formel I, die an einen Füllstoff adsorbiert sind, als Stabilisator in Überzügen bringt den zusätzlichen Vorteil mit sich, daß der Delaminierung, d. h. dem Abblättern des Überzuges vom Substrat, vorgebeugt wird. Dieser Vorteil kommt insbesondere bei metallischen Substraten zum tragen, auch bei Mehrschichtsystemen auf metallischen Substraten.

Als Bindemittel (Komponente A) kommen prinzipiell alle in der Technik gebräuchlichen in Betracht, beispielsweise solche, wie sie beschrieben sind in Ullmann's Encyclopedia of Industrial Chemistry, 5. Ed., Vol. A 18,
Seiten 368-426, VCH Verlagsgesellschaft, Weinheim 1991. Allgemein handelt es sich um ein filmbildendes Bindemittel basierend auf einem thermoplastischen oder thermohärtbaren Harz, vorwiegend auf einem thermohärtbaren Harz. Beispiele hierfür sind Alkyd-, Acryl-, Polyester-, Phenol-, Melamin-, Epoxid-, Polyurethanharze und deren Gemische.

Komponente A kann ein kalt aushärtbares oder ein heiß aushärtbares Bindemittel sein, wobei die Zugabe eines Härtungskatalysators vorteilhaft sein kann. Geeignete Katalysatoren, die die Aushärtung des Bindemittels beschleunigen, sind beispielsweise beschrieben in Ullmann's Encyclopedia of Industrial Chemistry, Vol. A 18, S. 469, VCH Verlagsgesellschaft, Weinheim 1991.

Bevorzugt sind Überzugsmittel, worin die Komponente A ein Bindemittel aus einem funktionellen Acrylatharz und einem Vernetzer ist.

Beispiele von Überzugsmitteln mit speziellen Bindemitteln sind:
1. Lacke auf Basis von kalt- oder heiß-vernetzbaren Alkyd-, Acrylat-, Polyester-, Epoxidoder Melaminharzen oder Mischungen solcher Harze, gegebenenfalls mit Zusatz eines Härtungskatalysators;
2. Zweikomponenten-Polyurethanlacke auf Basis von hydroxylgruppenhaltigen Acrylat-, Polyester- oder Polyetherharzen und aliphatischen oder aromatischen Isocyanaten, Isocyanuraten oder Polyisocyanaten;
3. Einkomponenten-Polyurethanlacke auf Basis von blockierten Isocyanaten, Isocyanuraten oder Polyisocyanaten, die während des Einbrennens deblockiert werden;
4. Einkomponenten-Polyurethanlacke auf Basis von aliphatischen oder aromatischen Urethanen oder Polyurethanen und hydroxylgruppenhaltigen Acrylat-, Polyester- oder Polyetherharzen;
5. Einkomponenten-Polyurethanlacke auf Basis von aliphatischen oder aromatischen Urethanen oder Polyurethanen mit freien Amingruppen in der Urethanstruktur und Melaminharzen oder Polyetherharzen, gegebenenfalls mit Zusatz eines Härtungskatalysators;
6. Zweikomponentenlacke auf Basis von (Poly)ketiminen und aliphatischen oder aromatischen Isocyanaten, Isocyanuraten oder Polyisocyanaten;
7. Zweikomponentenlacke auf Basis von (Poly)ketiminen und einem ungesättigten Acrylatharz oder einem Polyacetoacetatharz oder einem Methacrylamidoglykolatmethylester;
8. Zweikomponentenlacke auf Basis von carboxyl- oder aminogruppenhaltigen Polyacrylaten und Polyepoxiden;
9. Zweikomponentenlacke auf Basis von anhydridgruppenhaltigen Acrylatharzen und einer Polyhydroxy- oder Polyaminokomponente;
10. Zweikomponentenlacke auf Basis von acrylathaltigen Anhydriden und Polyepoxiden;
11. Zweikomponentenlacke auf Basis von (Poly)oxazolinen und anhydridgruppenhaltigen Acrylatharzen oder ungesättigten Acrylatharzen oder aliphatischen oder aromatischen Isocyanaten, Isocyanuraten oder Polyisocyanaten;
12. Zweikomponentenlacke auf Basis von ungesättigten Polyacrylaten und Polymalonaten;
13. thermoplastische Polyacrylatlacke auf Basis von thermoplastischen Acrylatharzen oder fremdvernetzenden Acrylatharzen in Kombination mit veretherten Melaminharzen;
14. Lacksysteme auf Basis von siloxanmodifizierten oder fluormodifizierten Acrylatharzen.

Das Überzugsmittel kann außer den Komponenten A und B gegebenenfalls weitere Komponenten enthalten, z. B. Lösungsmittel, Pigmente, Farbstoffe, Weichmacher, Stabilisatoren, Thixotropiemittel, Trocknungskatalysatoren und/oder Verlaufhilfsmittel. Mögliche Komponenten sind beispielsweise solche, wie sie in Ullmann's Encyclopedia of Industrial Chemistry, 5. Ed., Vol. A 18, Seiten 429-471, VCH Verlagsgesellschaft, Weinheim 1991 beschrieben sind.

Mögliche Trocknungskatalysatoren beziehungsweise Härtungskatalysatoren sind beispielsweise organische Metallverbindungen, Amine, aminogruppenhaltige Harze oder/und Phosphine. Organische Metallverbindungen sind z. B. Metallcarboxylate, insbesondere solche der Metalle Pb, Mn, Co, Zn, Zr oder Cu, oder Metallchelate, insbesondere solche der Metalle Al, Ti oder Zr oder Organometallverbindungen wie z. B. Organozinnverbindungen.

Beispiele für Metallcarboxylate sind die Stearate von Pb, Mn oder Zn, die Octoate von Co, Zn oder Cu, die Naphthenate von Mn und Co oder die entsprechenden Linoleate, Resinate oder Tallate.

Beispiele für Metallchelate sind die Aluminium-, Titan- oder Zirkonium-Chelate von Acetylaceton, Ethylacetylacetat, Salicylaldehyd, Salicylaldoxim, o-Hydroxyacetophenon oder Ethyl-trifluoracetylacetat und die Alkoxide dieser Metalle.

Beispiele für Organozinnverbindungen sind Dibutylzinnoxid, Dibutylzinn-dilaurat oder Dibutylzinn-dioctoat.

Beispiele für Amine sind vor allem tertiäre Amine, wie z.B. Tributylamin, Triethanolamin, N-Methyl-diethanolamin, N-Dimethylethanolamin, N-Ethylmorpholin, N-Methylmorpholin oder Diazabicyclooctan (Triethylendiamin) sowie deren Salze. Weitere Beispiele sind quaternäre Ammoniumsalze, wie z. B. Trimethylbenzylammoniumchlorid.

Aminogruppenhaltige Harze sind gleichzeitig Bindemittel und Härtungskatalysator. Beispiel hierfür sind aminogruppenhaltige Acrylat-Copolymere.

Als Härtungskatalysator können auch Phosphine verwendet werden, wie z. B. Triphenylphosphin.

Bei den erfindungsgemäßen Überzugsmitteln kann es sich auch um strahlen-härtbare Überzugsmittel handeln. In diesem Fall besteht das Bindemittel im wesentlichen aus monomeren oder oligomeren Verbindungen mit ethylenisch ungesättigten Bindungen (Präpolymeren), die nach der Applikation durch aktinische Strahlung gehärtet, d. h. in eine vernetzte, hochmolekulare Form überführt werden. Handelt es sich um ein UV-härtendes System, enthält dies in der Regel zusätzlich einen Photoinitiator. Entsprechende Systeme sind in der oben genannten Publikation, Ullmann's Encyclopedia of Industrial Chemistry, 5. Ed., Vol. A 18, Seiten 451-453, beschrieben.

Die erfindungsgemäßen Überzugsmittel können auf beliebige Substrate aufgebracht werden, beispielsweise auf Metall, Holz, Kunststoff oder keramische Materialien. Vorzugsweise werden sie beim Lackieren von Automobilen als Decklack verwendet. Besteht der Decklack aus zwei Schichten, wovon die untere Schicht pigmentiert ist und die obere Schicht nicht pigmentiert ist, so kann das erfindungsgemäße Überzugsmittel für die obere oder die untere Schicht oder für beide Schichten verwendet werden, vorzugsweise jedoch für die obere Schicht.

Die erfindungsgemäßen Überzugsmittel können auf die Substrate nach den üblichen Verfahren aufgebracht werden, beispielsweise durch Streichen, Besprühen, Gießen, Tauchen oder Elektrophorese; s. a. Ullmann's Encyclopedia of Industrial Chemistry, 5. Ed., Vol. A 18, Seiten 491-500.

Die Härtung der Überzüge kann - je nach dem Bindemittelsystem - bei Raumtemperatur oder durch Erwärmen erfolgen. Vorzugsweise härtet man die Überzüge bei 50-150°C, Pulverlacke auch bei höheren Temperaturen.

Die erfindungsgemäß erhaltenen Überzüge weisen eine hervorragende Beständigkeit gegen schädigende Einflüsse von Licht, Sauerstoff und Wärme auf; insbesondere ist auf die gute Licht- und Witterungsbeständigkeit der so erhaltenen Überzüge, beispielsweise Lacke, hinzuweisen.

Gegenstand der Erfindung ist daher auch ein Überzug, besonders ein Lack, der durch Zusatz einer Verbindung der Formel I, sowie deren erfindungsgemässen Homo-, Co- und Terpolymeren sowie die Verbindungen, erhältlich durch Umsetzung einer oder mehrerer Verbindungen der Formel I mit mindestens einer weiteren nicht der Formel I entsprechenden Mono-Glycidylverbindung als auch Verbindungen, erhältlich durch Umsetzung einer oder mehrerer Verbindungen der Formel I mit mindestens einem Amin oder einer Carbonsäure oder einem Phenol oder Dicarbonsäureanhydrid oder Alkoholat sowie Verbindungen der Formel I, die an einen Füllstoff adsorbiert sind, gegen schädigende Einflüsse von Licht, Sauerstoff und Wärme stabilisiert ist. Der Lack ist vorzugsweise ein Decklack für Automobile. Die Erfindung beinhaltet weiterhin ein Verfahren zum Stabilisieren eines Überzuges auf Basis organischer Polymere gegen Schädigung durch Licht, Sauerstoff und/oder Wärme, dadurch gekennzeichnet, daß man dem Überzugsmittel eine Verbindung der Formel I, sowie deren erfindungsgemässen Homo-, Co- und Terpolymeren sowie die Verbindungen, erhältlich durch Umsetzung einer oder mehrerer Verbindungen der Formel I mit mindestens einer weiteren nicht der Formel I entsprechenden Mono-Glycidylverbindung als auch Verbindungen, erhältlich durch Umsetzung einer oder mehrerer Verbindungen der Formel I mit mindestens einem Amin oder einer Carbonsäure oder einem Phenol oder Dicarbonsäureanhydrid oder Alkoholat sowie Verbindungen der Formel I, die an einen Füllstoff adsorbiert sind, beimischt, sowie die Verwendung Verbindungen der Formel I, sowie deren erfindungsgemässen Homo-, Co- und Terpolymeren sowie die Verbindungen, erhältlich durch Umsetzung einer oder mehrerer Verbindungen der Formel I mit mindestens einer weiteren nicht der Formel I entsprechenden Mono-Glycidylverbindung als auch Verbindungen, erhältlich durch Umsetzung einer oder mehrerer Verbindungen der Formel I mit mindestens einem Amin oder einer Carbonsäure oder einem Phenol oder Dicarbonsäureanhydrid oder Alkoholat sowie Verbindungen der Formel I, die an einen Füllstoff adsorbiert sind, in Überzugsmitteln als Stabilisatoren gegen Schädigung durch Licht, Sauerstoff und/oder Wärme.

Die Überzugsmittel können ein organisches Lösungsmittel oder Lösungsmittel-gemisch enthalten, in dem das Bindemittel löslich ist. Das Überzugsmittel kann aber auch eine wässrige Lösung oder Dispersion sein. Das Vehikel kann auch ein Gemisch eines organischen Lösungmittels und Wasser sein. Das Überzugsmittel kann auch ein feststoffreicher Lack (high solids Lack) sein oder kann lösungsmittelfrei sein (z. B. Pulverlack). Pulverlacke sind beispielsweise solche, wie sie in Ullmann's Encyclopedia of Industrial Chemistry, 5. Ed., A 18, Seiten 438-444 beschrieben sind. Der Pulverlack kann auch in Form eines Pulver-Slurry, d. h. einer Dispersion des Pulvers bevorzugt in Wasser vorliegen.

Die Pigmente können anorganische, organische oder metallische Pigmente sein. Vorzugsweise enthalten die erfindungsgemäßen Überzugsmittel keine Pigmente und werden als Klarlack verwendet.

Ebenfalls bevorzugt ist der Einsatz des Überzugsmittels als Decklack für Anwendungen in der Automobilindustrie, besonders als pigmentierte oder unpigmentierte Deckschicht des Lackes. Die Verwendung für darunter liegende Schichten ist jedoch auch möglich.

Weitere mit den erfindungsgemässen Verbindungen zu stabilisierende Materialien sind photographische Materialien. Unter solchen sind insbesondere solche zu verstehen, die in Research Disclosure 1990, 31429 (Seiten 474-480) für die photographische Reproduktion und andere Reproduktionstechniken beschrieben sind.

Allgemein werden die Verbindungen der Formel I dem zu stabilisierenden Material in Mengen von 0,01 bis 10%, bevorzugt 0,01 bis 5%, insbesondere 0,01 bis 2%, bezogen auf das Gesamtgewicht der stabilisierten Zusammensetzung, zugesetzt. Besonders bevorzugt ist der Einsatz der erfindungsgemäßen Verbindungen in Mengen von 0,05 bis 1,5%, vor allem 0,1 bis 0,5%.

Die Einarbeitung in die Materialien kann beispielsweise durch Einmischen oder Aufbringen der Verbindungen der Formel I und gegebenenfalls weiterer Additive nach den in der Technik üblichen Methoden erfolgen. Handelt es sich um Polymere, insbesondere synthetische Polymere, kann die Einarbeitung vor oder während der Formgebung, oder durch Aufbringen der gelösten oder dispergierten Verbindung auf das Polymere, gegebenenfalls unter nachträglichem Verdunsten des Lösungsmittels erfolgen. Im Fall von Elastomeren können diese auch als Latices stabilisiert werden. Eine weitere Möglichkeit der Einarbeitung der Verbindungen der Formel I in Polymere besteht in deren Zugabe vor, während oder unmittelbar nach der Polymerisation der entsprechenden Monomeren bzw. vor der Vernetzung. Die Verbindung der Formel I kann dabei als solche, aber auch in encapsulierter Form (z.B in Wachsen, Ölen oder Polymeren) zugesetzt werden. Im Falle der Zugabe vor oder während der Polymerisation können die Verbindungen der Formel I auch als Regulator für die Kettenlänge der Polymere (Kettenabbrecher) wirken.

Die Verbindungen der Formel I können auch in Form eines Masterbatches, der diese Verbindung beispielsweise in einer Konzentration von 2,5 bis 25 Gew.-% enthält, den zu stabilisierenden Kunststoffen zugesetzt werden.

Zweckmäßig kann die Einarbeitung der Verbindungen der Formel I nach folgenden Methoden erfolgen:
- als Emulsion oder Dispersion (z.B. zu Latices oder Emulsionspolymeren),
- als Trockenmischung während des Vermischens von Zusatzkomponenten oder Polymermischungen,
- durch direktes Zugeben in die Verarbeitungsapparatur (z.B. Extruder, Innenmischer usw.),
- als Lösung oder Schmelze.

Erfindungsgemäße Polymerzusammensetzungen können in verschiedener Form angewendet bzw. zu verschiedenen Produkten verarbeitet werden, z.B. als (zu) Folien, Fasern, Bändchen, Formmassen, Profilen, oder als Bindemittel für Lacke, Klebstoffe oder Kitte.

Neben den Verbindungen der Formel I, sowie deren erfindungsgemässen Homo-, Co- und Terpolymeren sowie den Verbindungen, erhältlich durch Umsetzung einer oder mehrerer Verbindungen der Formel I mit mindestens einer weiteren nicht der Formel I entsprechenden Mono-Glycidylverbindung als auch Verbindungen, erhältlich durch Umsetzung einer oder mehrerer Verbindungen der Formel I mit mindestens einem Amin oder einer Carbonsäure oder einem Phenol oder Dicarbonsäureanhydrid oder Alkoholat sowie Verbindungen der Formel I, die an einen Füllstoff adsorbiert sind, können die erfindungsgemäßen Zusammensetzungen als zusätzliche Komponente (c) ein oder mehrere herkömmliche Additive enthalten, wie beispielsweise die folgenden:
1. Antioxidantien
   1.1. Alkylierte Monophenole, z.B. 2,6-Di-tert-butyl-4-methylphenol, 2-Butyl-4,6-dimethylphenol, 2,6-Di-tert-butyl-4-ethylphenol, 2,6-Di-tert-butyl-4-n-butylphenol, 2,6-Di-tert-butyl-4-iso-butylphenol, 2,6-Di-cyclopentyl-4-methylphenol, 2-(α-Methylcyclohexyl)-4,6-dimethylphenol, 2,6-Di-octadecyl-4-methylphenol, 2,4,6-Tri-cyclohexylphenol, 2,6-Di-tert-butyl-4-methoxymethylphenol, lineare oder in der Seitenkette verzweigte Nonylphenole wie z.B. 2,6-Di-nonyl-4-methylphenol, 2,4-Dimethyl-6-(1'-methyl-undec-1'-yl)-phenol, 2,4-Dimethyl-6-(1'-methyl-heptadec-1'-yl)-phenol, 2,4-Dimethyl-6-(1'-methyl-tridec-1'-yl)-phenol und Mischungen davon.
   1.2. Alkylthiomethylphenole, z.B. 2,4-Di-octylthiomethyl-6-tert-butylphenol, 2,4-Di-octylthiomethyl-6-methylphenol, 2,4-Di-octylthiomethyl-6-ethylphenol, 2,6-Di-dodecylthiomethyl-4nonylphenol.
   1.3. Hydrochinone und alkylierte Hydrochinone, z.B. 2,6-Di-tert-butyl-4-methoxyphenol, 2,5Di-tert-butyl-hydrochinon, 2,5-Di-tert-amyl-hydrochinon, 2,6-Diphenyl-4-octadecyloxyphenol, 2,6-Di-tert-butyl-hydrochinon, 2,5-Di-tert-butyl-4-hydroxyanisol, 3,5-Di-tert-butyl-4-hydroxyanisol, 3,5-Di-tert-butyl-4-hydroxyphenyl-stearat, Bis(3,5-di-tert-butyl-4-hydroxyphenyl)adipat.
   1.4. Tocopherole, z.B. α-Tocopherol, β-Tocopherol, γ-Tocopherol, δ-Tocopherol und Mischungen davon (Vitamin E).
   1.5. Hydroxylierte Thiodiphenylether, z.B. 2,2'-Thio-bis(6-tert-butyl-4-methylphenol), 2,2'Thio-bis(4-octylphenol), 4,4'-Thio-bis(6-tert-butyl-3-methylphenol), 4,4'-Thio-bis-(6-tert-butyl2-methylphenol), 4,4'-Thio-bis(3,6-di-sec.-amylphenol), 4,4'-Bis(2,6-dimethyl-4-hydroxyphenyl)-disulfid.
   1.6. Alkyliden-Bisphenole, z.B. 2,2'-Methylen-bis(6-tert-butyl-4-methylphenol), 2,2'-Methylenbis(6-tert-butyl-4-ethylphenol), 2,2'-Methylen-bis[4-methyl-6-(a-methylcyclohexyl)-phenol], 2,2'-Methylen-bis(4-methyl-6-cyclohexylphenol), 2,2'-Methylen-bis(6-nonyl-4-methylphenol), 2,2'-Methylen-bis(4,6-di-tert-butylphenol), 2,2'-Ethyliden-bis(4,6-di-tert-butylphenol), 2,2'Ethyliden-bis(6-tert-butyl-4-isobutylphenol), 2,2'-Methylen-bis[6-(a-methylbenzyl)-4-nonylphenol], 2,2'-Methylen-bis[6-(a,a-dimethylbenzyl)-4-nonylphenol], 4,4'-Methylen-bis(2,6-ditert-butylphenol), 4,4'-Methylen-bis(6-tert-butyl-2-methylphenol), 1,1-Bis(5-tert-butyl-4hydroxy-2-methylphenyl)-butan, 2,6-Bis(3-tert-butyl-5-methyl-2-hydroxybenzyl)-4-methylphenol, 1,1,3-Tris(5-tert-butyl-4-hydroxy-2-methylphenyl)-butan, 1,1-Bis(5-tert-butyl-4hydroxy-2-methyl-phenyl)-3-n-dodecylmercaptobutan, Ethylenglycol-bis[3,3-bis(3'-tert-butyl4'-hydroxyphenyl)-butyrat], Bis(3-tert-butyl-4-hydroxy-5-methyl-phenyl)-dicyclopentadien, Bis[2-(3'-tert-butyl-2'-hydroxy-5'-methyl-benzyl)-6-tert-butyl-4-methyl-phenyl]-terephthalat, 1,1-Bis(3,5-dimethyl-2-hydroxyphenyl)-butan, 2,2-Bis(3,5-di-tert-butyl-4-hydroxyphenyl)propan, 2,2-Bis(5-tert-butyl-4-hydroxy-2-methylphenyl)-4-n-dodecylmercapto-butan, 1,1,5,5Tetra-(5-tert-butyl-4-hydroxy-2-methylphenyl)-pentan.
   1.7. O-, N- und S-Benzylverbindungen, z.B. 3,5,3',5'-Tetra-tert-butyl-4,4'-dihydroxydibenzylether, Octadecyl-4-hydroxy-3,5-dimethylbenzyl-mercaptoacetat, Tridecyl-4-hydroxy-3,5-ditert-butylbenzyl-mercaptoacetat, Tris(3,5-di-tert-butyl-4-hydroxybenzyl)-amin, Bis(4-tertbutyl-3-hydroxy-2,6-dimethylbenzyl)-dithioterephthalat, Bis(3,5-di-tert-butyl-4-hydroxybenzyl)-sulfid, Isooctyl-3,5-di-tert-butyl-4-hydroxybenzyl-mercaptoacetat.
   1.8. Hydroxybenzylierte Malonate, z.B. Dioctadecyl-2,2-bis(3,5-di-tert-butyl-2-hydroxybenzyl)-malonat, Di-octadecyl-2-(3-tert-butyl-4-hydroxy-5-methylbenzyl)-malonat, Di-dodecylmercaptoethyl-2,2-bis(3,5-di-tert-butyl-4-hydroxybenzyl)-malonat, Di-[4-(1,1,3,3tetramethylbutyl)-phenyl]-2,2-bis(3,5-di-tert-butyl-4-hydroxybenzyl)-malonat.
   1.9. Hydroxybenzyl-Aromaten, z. B. 1 ,3,5-Tris(3,5-di-tert-butyl-4-hydroxybenzyl)-2,4,6-trimethylbenzol, 1,4-Bis(3,5-di-tert-butyl-4-hydroxybenzyl)-2,3,5,6-tetramethylbenzol, 2,4,6Tris(3,5-di-tert-butyl-4-hydroxybenzyl)-phenol.
   1.10. Triazinverbindungen, z.B. 2,4-Bis-octylmercapto-6-(3,5-di-tert-butyl-4-hydroxyanilino)1,3,5-triazin, 2-Octylmercapto-4,6-bis(3,5-di-tert-butyl-4-hydroxyanilino)-1 ,3,5-triazin, 2Octylmercapto-4,6-bis(3,5-di-tert-butyl-4-hydroxyphenoxy)-1,3,5-triazin, 2,4,6-Tris(3,5-ditert-butyl-4-hydroxyphenoxy)-1 ,2,3-triazin, 1,3,5-Tris(3,5-di-tert-butyl-4-hydroxybenzyl)isocyanurat, 1,3,5-Tris(4-tert-butyl-3-hydroxy-2,6-dimethylbenzyl)-isocyanurat, 2,4,6Tris(3, 5-di-tert-butyl-4-hydroxyphenylethyl) -1,3,5-triazin, 1 ,3,5-Tris(3,5-di-tert-butyl-4hydroxyphenylpropionyl)-hexahydro-1,3,5-triazin, 1,3,5-Tris(3,5-dicyclohexyl-4-hydroxybenzyl)-isocyanurat.
   1.11. Benzylphosphonate, z.B. Dimethyl-2,5-di-tert-butyl-4-hydroxybenzylphosphonat, Diethyl-3,5-di-tert-butyl-4-hydroxybenzylphosphonat, Dioctadecyl-3,5-di-tert-butyl-4-hydroxybenzylphosphonat, Dioctadecyl-5-tert-butyl-4-hydroxy-3-methylbenzylphosphonat, Ca-Salz des 3,5-Di-tert-butyl-4-hydroxybenzyl-phosphonsäure-monoethylesters.
   1.12. Acylaminophenole, z.B. 4-Hydroxy-laurinsäureanilid, 4-Hydroxystearinsäureanilid, N(3,5-di-tert-butyl-4-hydroxyphenyl)-carbaminsäureoctylester.
   1.13. Ester der β-(3,5-Di-tert-butyl-4-hydroxyphenyl)-propionsäure mit ein- oder mehrwertigen Alkoholen, wie z.B. mit Methanol, Ethanol, n-Octanol, i-Octanol, Octadecanol, 1,6Hexandiol, 1,9-Nonandiol, Ethylenglycol, 1,2-Propandiol, Neopentylglycol, Thiodiethylenglycol, Diethylenglycol, Triethylenglycol, Pentaerythrit, Tris(hydroxyethyl)-isocyanurat, N,N'-Bis(hydroxyethyl)-oxalsäurediamid, 3-Thiaundecanol, 3-Thiapentadecanol, Trimethylhexandiol, Trimethylolpropan, 4-Hydroxymethyl-1-phospha-2,6,7-trioxabicyclo-[2.2.2]-octan.
   1.14. Ester der β-(5-tert-Butyl-4-hydroxy-3-methylphenyl)-propionsäure mit ein- oder mehrwertigen Alkoholen, wie z.B. mit Methanol, Ethanol, n-Octanol, i-Octanol, Octadecanol, 1,6Hexandiol, 1,9-Nonandiol, Ethylenglycol, 1,2-Propandiol, Neopentylglycol, Thiodiethylenglycol, Diethylenglycol, Triethylenglycol, Pentaerythrit, Tris(hydroxyethyl)-isocyanurat, N,N'Bis(hydroxyethyl)-oxalsäurediamid, 3-Thiaundecanol, 3-Thiapentadecanol, Trimethylhexandiol, Trimethylolpropan, 4-Hydroxymethyl-1-phospha-2,6,7-trioxabicyclo-[2.2.2]-octan.
   1.15. Ester der β-(3,5-Dicyclohexyl-4-hydroxyphenyl-propionsäure mit ein- oder mehrwertigen Alkoholen, wie z.B. mit Methanol, Ethanol, Octanol, Octadecanol, 1,6-Hexandiol, 1,9Nonandiol, Ethylenglycol, 1,2-Propandiol, Neopentylglycol, Thiodiethylenglycol, Diethylenglycol, Triethylenglycol, Pentaerythrit, Tris(hydroxyethyl)-isocyanurat, N,N'-Bis(hydroxyethyl)-oxalsäurediamid, 3-Thiaundecanol, 3-Thiapentadecanol, Trimethylhexandiol, Trimethylolpropan, 4-Hydroxymethyl-1-phospha-2,6,7-trioxabicyclo-[2.2.2]-octan.
   1.16. Ester der 3,5-Di-tert-butyl-4-hydroxyphenylessigsäure mit ein- oder mehrwertigen Alkoholen, wie z.B. mit Methanol, Ethanol, Octanol, Octadecanol, 1,6-Hexandiol, 1,9Nonandiol, Ethylenglycol, 1,2-Propandiol, Neopentylglycol, Thiodiethylenglycol, Diethylenglycol, Triethylenglycol, Pentaerythrit, Tris(hydroxyethyl)-isocyanurat, N,N'-Bis(hydroxyethyl)-oxalsäurediamid, 3-Thiaundecanol, 3-Thiapentadecanol, Trimethylhexandiol, Trimethylolpropan, 4-Hydroxymethyl-1-phospha-2,6,-trioxabicyclo-[2.2.2]-octan.
   1.17. Amide der β-(3,5-Di-tert-buyl-4-hydroxyphenyl)-propionsäure, wie z.B. N,N'-Bis(3,5-ditert-butyl-4-hydroxyphenylpropionyl)-hexamethylendiamin, N,N'-Bis(3,5-di-tert-butyl-4hydroxyphenylpropionyl)-trimethylendiamin, N,N'-Bis(3,5-di-tert-butyl-4-hydroxyphenylpropionyl)-hydrazin.
   1.18. Ascorbinsäure (Vitamin C).
   1.19. Aminische Antioxidantien, wie z.B. N,N'-Di-isopropyl-p-phenylendiamin, N,N'-Di-secbutyl-p-phenylendiamin, N,N'-Bis(1,4-dimethyl-pentyl)-p-phenylendiamin, N,N'-Bis(1-ethyl-3-methyl-pentyl)-p-phenylendiamin, N,N'-Bis(1-methyl-heptyl)-p-phenylendiamin, N,N'Dicyclohexyl-p-phenylendiamin, N,N'-Diphenyl-p-phenylendiamin, N,N'-Di-(naphthyl-2)-pphenylendiamin, N-lsopropyl-N'-phenyl-p-phenylendiamin, N-(1,3-Dimethyl-butyl)-N'-phenylp-phenylendiamin, N-(1-Methyl-heptyl)-N'-phenyl-p-phenylendiamin, N-Cyclohexyl-N'phenyl-p-phenylendiamin, 4-(p-Toluol-sulfonamido)-diphenylamin, N,N'-Dimethyl-N,N'-disec-butyl-p-phenylendiamin, Diphenylamin, N-Allyldiphenylamin, 4-lsopropoxy-diphenylamin, N-Phenyl-1-naphthylamin, N-(4-tert-Octylphenyl)-1-naphthylamin, N-Phenyl-2-naphthylamin, octyliertes Diphenylamin, z.B. p,p'-Di-tert-octyldiphenylamin, 4-n-Butylaminophenol, 4-Butyrylamino-phenol, 4-Nonanoylamino-phenol, 4-Dodecanoylamino-phenol, 4-Octadecanoylamino-phenol, Di-(4-methoxyphenyl)-amin, 2,6-Di-tert-butyl-4-dimethylaminomethyl-phenol, 2,4'-Diamino-diphenylmethan, 4,4'-Diamino-diphenylmethan, N,N,N',N'Tetramethyl-4,4'-diamino-diphenylmethan, 1,2-Di-[(2-methyl-phenyl)-amino]-ethan, 1,2-Di(phenylamino)-propan, (o-Tolyl)-biguanid, Di-[4-(1',3'-dimethyl-butyl)-phenyl]amin, tert-octyliertes N-Phenyl-1-naphthylamin, Gemisch aus mono- und dialkylierten tert-Butyl/tert-Octyldiphenylaminen, Gemisch aus mono- und dialkylierten Nonyldiphenylaminen, Gemisch aus mono- und dialkylierten Dodecyldiphenylaminen, Gemisch aus mono- und dialkylierten Isopropyl/lsohexyl-diphenylaminen, Gemische aus mono- und dialkylierten tertButyldiphenylaminen, 2,3-Dihydro-3,3-dimethyl-4H-1,4-benzothiazin, Phenothiazin, Gemisch aus mono- und dialkylierten tert-Butyl/tert-Octyl-phenothiazinen, Gemisch aus monound dialkylierten tert-Octyl-phenothiazinen, N-Allylphenothiazin, N,N,N',N'-Tetraphenyl-1,4diaminobut-2-en, N,N-Bis-(2,2,6,6-tetramethyl-piperidin-4-yl-hexamethylendiamin, Bis(2,2,6,6-tetramethylpiperidin-4-yl)-sebacat, 2,2,6,6-Tetramethylpiperidin-4-on, 2,2,6,6-Tetramethylpiperidin-4-ol.

2. UV-Absorber und Lichtschutzmittel
   2.1. 2-(2'-Hydroxyphenyl)-benzotriazole, wie z.B. 2-(2'-Hydroxy-5'-methylphenyl)-benzotriazol, 2-(3',5'-Di-tert-butyl-2'-hydroxyphenyl)-benzotriazol, 2-(5'-tert-Butyl-2'-hydroxyphenyl)benzotriazol, 2-(2'-Hydroxy-5'-(1,1 ,3,3-tetramethylbutyl)phenyl)-benzotriazol, 2-(3',5'-Di-tertbutyl-2'-hydroxyphenyl)-5-chlor-benzotriazol, 2-(3'-tert-Butyl- 2'-hydroxy-5'-methylphenyl)-5chlor-benzotriazol, 2-(3'-sec-Butyl-5'-tert-butyl-2'-hydroxyphenyl)-benzotriazol, 2-(2'Hydroxy-4'-octoxyphenyl)-benzotriazol, 2-(3',5'-Di-tert-amyl-2'-hydroxyphenyl)-benzotriazol, 2-(3',5'-Bis(a,a-dimethylbenzyl)-2'-hydroxyphenyl)-benzotriazol, Mischung aus 2-(3'-tertButyl-2'-hydroxy-5'-(2-octyloxycarbonylethyl)phenyl)-5-chlor-benzotriazol, 2-(3'-tert-Butyl-5'[2-(2-ethylhexyloxy)-carbonylethyl]-2'-hydroxyphenyl)-5-chlor-benzotriazol, 2-(3'-tert-Butyl-2'hydroxy-5'-(2-methoxycarbonylethyl)phenyl)-5-chlor-benzotriazol, 2-(3'-tert-Butyl-2'-hydroxy5'- (2-methoxycarbonylethyl)phenyl)-benzotriazol, 2-(3'-tert-Butyl-2'-hydroxy-5'-(2-octyloxycarbonylethyl)phenyl)-benzotriazol, 2-(3'-tert-Butyl-5'-[2-(2-ethylhexyloxy)carbonylethyl]-2'hydroxyphenyl)-benzotriazol, 2-(3'-Dodecyl-2'-hydroxy-5'-methylphenyl)-benzotriazol, und 2(3'-tert-Butyl-2'-hydroxy-5'-(2-isooctyloxycarbonylethyl)phenyl-benzotriazol, 2,2'-Methylenbis[4-(1,1 ,3,3-tetramethylbutyl)-6-benzotriazol-2-yl-phenol]; Umesterungsprodukt von 2-[3'tert-Butyl-5'-(2-methoxycarbonylethyl)-2'-hydroxy-phenyl]-benzotriazol mit Polyethylenglycol 300;mit R = 3'-tert-Butyl-4'-hydroxy-5'-2H-benzotriazol-2-yl-phenyl.
   2.2. 2-Hydroxybenzophenone, wie z.B. das 4-Hydroxy-, 4-Methoxy-, 4-Octoxy-, 4-Decyloxy-, 4-Dodecyloxy-, 4-Benzyloxy-, 4,2',4'-Trihydroxy-, 2'-Hydroxy-4,4'-dimethoxy-Derivat.
   2.3. Ester von gegebenenfalls substituierten Benzoesäuren, wie z.B. 4-tert-Butyl-phenylsalicylat, Phenylsalicylat, Octylphenyl-salicylat, Dibenzoylresorcin, Bis(4-tert-butylbenzoyl)resorcin, Benzoylresorcin, 3,5-Di-tert-butyl-4-hydroxybenzoesäure-2,4-di-tert-butylphenylester, 3,5-Di-tert-butyl-4-hydroxybenzoesäurehexadecylester, 3,5-Di-tert-butyl-4hydroxybenzoesäure-octadecylester, 3,5-Di-tert-butyl-4-hydroxybenzoesäure-2-methyl-4,6di-tert-butylphenylester.
   2.4. Acrylate, wie z.B. α-Cyan-β,β-diphenylacrylsäure-ethylester bzw. -isooctylester, αCarbomethoxy-zimtsäuremethylester, α-Cyano-β-methyl-p-methoxy-zimtsäuremethylester bzw. -butylester, α-Carbomethoxy-p-methoxy-zimtsäure-methylester, N-(β-Carbomethoxy-βcyanovinyl)-2-methyl-indolin.
   2.5. Nickelverbindungen, wie z.B. Nickelkomplexe des 2,2'-Thio-bis[4-(1,1,3,3-tetramethylbutyl)-phenols], wie der 1:1- oder der 1:2-Komplex, gegebenenfalls mit zusätzlichen Liganden, wie n-Butylamin, Triethanolamin oder N-Cyclohexyl-diethanolamin, Nickeldibutyl dithiocarbamat, Nickelsalze von 4-Hydroxy-3,5-di-tert-butylbenzylphosphonsäure-monoalkylestern, wie vom Methyl- oder Ethylester, Nickelkomplexe von Ketoximen, wie von 2-Hydroxy-4-methyl-phenyl-undecylketoxim, Nickelkomplexe des 1-Phenyl-4-lauroyl-5hydroxy-pyrazols, gegebenenfalls mit zusätzlichen Liganden.
   2.6. Sterisch gehinderte Amine, wie z.B. Bis(2,2,6,6-tetramethyl-piperidin-4-yl)-sebacat, Bis(2,2,6,6-tetramethyl-piperidin-4-yl)-succinat, Bis(1 ,2,2,6,6-pentamethylpiperidin-4-yl)sebacat, Bis(1-octyloxy-2,2,6,6-tetramethylpiperidin-4-yl)-sebacat, n-Butyl-3,5-di-tert-butyl-4hydroxybenzyl-malonsäure-bis(1,2, 2,6,6-pentamethylpiperidyl)-ester, Kondensationsprodukt aus 1-Hydroxyethyl-2,2,6,6-tetramethyl-4-hydroxypiperidin und Bernsteinsäure, Kondensationsprodukt aus N,N'-Bis(2,2,6,6-Tetramethyl-4-piperidyl)-hexamethylendiamin und 4-tert-Octylamino-2,6-dichlor-1,3,5-s-triazin, Tris(2,2,6,6-tetramethyl-4-piperidyl)-nitrilotriacetat, Tetrakis(2,2,6,6-tetramethyl-4-piperidyl)-1,2,3,4-butantetraoat, 1,1'-(1 ,2-Ethandiyl)bis(3,3,5,5-tetramethyl-piperazinon), 4-Benzoyl-2,2,6,6-tetramethylpiperidin, 4-Stearyloxy2,2,6,6-tetramethylpiperidin, Bis(1,2,2,6,6-pentamethylpiperidyl)-2-n-butyl-2-(2-hydroxy-3,5di-tert-butylbenzyl)-malonat, 3-n-Octyl-7,7,9,9-tetramethyl-1,3,8-triazaspiro[4.5]decan-2,4dion, Bis(1-octyloxy-2,2,6,6-tetramethylpiperidyl)-sebacat, Bis(1-octyloxy-2,2,6,6-tetramethylpiperidyl)-succinat, Kondensationsprodukt aus N,N'-Bis(2,2,6,6-tetramethyl-4-piperidyl)-hexamethylendiamin und 4-Morpholino-2,6-dichlor-1,3,5-triazin, Kondensationsprodukt aus 2-Chlor-4,6-di-(4-n-butylamino-2,2,6,6-tetramethylpiperidyl)-1,3,5-triazin und 1,2-Bis(3aminopropylamino)ethan, Kondensationsprodukt aus 2-Chlor-4,6-di-(4-n-butylamino1,2,2,6,6-pentamethylpiperidyl)-1,3,5-triazin und 1,2-Bis(3-aminopropylamino)-äthan, 8Acetyl-3-dodecyl-7,7,9,9-tetramethyl-1,3,8-triazaspiro[4.5]decan-2,4-dion, 3-Dodecyl-1(2,2,6,6-tetramethyl-4-piperidyl)pyrrolidin-2,5-dion, 3-Dodecyl-1 -(1 ,2,2,6,6-pentamethyl-4piperidyl)-pyrrolidin-2,5-dion, Gemisch von 4-Hexadecyloxy- und 4-Stearyloxy-2,2,6,6tetramethylpiperidin, Kondenstionsprodukt aus N,N'-Bis(2,2,6,6-tetramethyl-4-piperidyl)hexamethylendiamin und 4-Cyclohexylamino-2,6-dichlor-1,3,5-triazin, Kondensationsprodukt aus 1,2-Bis(3-aminopropylamino)-ethan und 2,4,6-trichlor-1,3,5-triazin sowie 4Butylamino-2,2,6,6-tetramethyl-piperidin (CAS Reg. No. [136504-96-6]); N-(2,2,6,6-tetramethyl-4-piperidyl)-n-dodecylsuccinimid, N-(1,2,2,6,6-pentamethyl-4-piperidyl)-n-dodecylsuccinimid, 2-Undecyl-7,7,9,9-tetramethyl-1-oxa-3,8-diaza-4-oxo-spiro[4,5]decan, Umsetzungsprodukt von 7,7,9,9-Tetramethyl-2-cycloundecyl-1-oxa-3,8-diaza-4-oxospiro[4,5]decan und Epichlorhydrin.
   2.7. Oxalsäurediamide, wie z.B. 4,4'-Di-octyloxy-oxanilid, 2,2'-Diethoxy-oxanilid, 2,2'-Dioctyloxy-5,5'-di-tert-butyl-oxanilid, 2,2'-Di-dodecyloxy-5,5'-di-tert-butyl-oxanilid, 2-Ethoxy-2'ethyl-oxanilid, N,N'-Bis(3-dimethylaminopropyl)-oxalamid, 2-Ethoxy-5-tert-butyl-2'-ethyloxanilid und dessen Gemisch mit 2-Ethoxy-2'-ethyl-5,4'-di-tert-butyl-oxanilid, Gemische von ound p-Methoxy- sowie von o- und p-Ethoxy-di-substituierten Oxaniliden.
   2.8. 2-(2-Hydroxyphenyl)-1,3,5-triazine, wie z.B. 2,4,6-Tris(2-hydroxy-4-octyloxyphenyl)1,3,5-triazin, 2-(2-Hydroxy-4-octyloxyphenyl)-4,6-bis(2,4-dimethylphenyl)-1,3,5-triazin, 2(2,4-Dihydroxyphenyl)-4,6-bis(2,4-dimethylphenyl)-1,3,5-triazin, 2,4-Bis(2-hydroxy-4-propyloxyphenyl)-6-(2,4-dimethylphenyl)-1,3,5-triazin, 2-(2-Hydroxy-4-octyloxyphenyl) -4,6-bis(4methylphenyl)-1,3,5-triazin, 2-(2-Hydroxy-4-dodecyloxyphenyl)-4,6-bis (2,4-dimethylphenyl)1,3,5-triazin, 2- (2-Hydroxy-4-tridecyloxyphenyl)-4,6-bis (2,4-dimethylphenyl)-1,3,5-triazin, 2[2-Hydroxy-4- (2-hydroxy-3-butyloxy-propyloxy) phenyl]-4,6-bis (2,4-dimethylphenyl)-1,3,5triazin, 2-[2-Hydroxy-4-(2-hydroxy-3-octyloxy-propyloxy)phenyl]-4,6-bis(2,4-dimethylphenyl)1,3,5-triazin, 2-[4-(dodecyloxy/tridecyloxy-2-hydroxypropoxy)-2-hydroxy-phenyl]-4,6-bis(2,4dimethylphenyl)-1,3,5-triazin, 2-[2-Hydroxy-4-(2-hydroxy-3-dodecyloxy-propoxy)phenyl]-4,6bis(2,4-dimethylphenyl)-1,3,5-triazin, 2-(2-Hydroxy-4-hexyloxy)phenyl-4,6-diphenyl-1,3,5triazin, 2-(2-Hydroxy-4-methoxyphenyl)-4,6-diphenyl-1,3,5-triazin, 2,4,6-Tris[2-hydroxy-4-(3butoxy-2-hydroxy-propoxy)phenyl]-1,3,5-triazin, 2-(2-Hydroxyphenyl)-4-(4-methoxyphenyl)6-phenyl-1,3,5-triazin.

3. Metalldesaktivatoren, wie z.B. N,N'-Diphenyloxalsäurediamid, N-Salicylal-N'-salicyloylhydrazin, N,N'-Bis(salicyloyl)-hydrazin, N,N'-Bis(3,5-di-tert-butyl-4-hydroxyphenylpropionyl)hydrazin, 3-Salicyloylamino-1,2,4-triazol, Bis(benzyliden)-oxalsäuredihydrazid, Oxanilid, Isophthalsäure-dihydrazid, Sebacinsäure-bis-phenylhydrazid, N,N'-Diacetyl-adipinsäuredihydrazid, N,N'-Bis-salicyloyl-oxalsäure-dihydrazid, N,N'-Bis-salicyloyl-thiopropionsäuredihydrazid.
4. Phosphite und Phosphonite, wie z.B. Triphenylphosphit, Diphenylalkylphosphite, Phenyldialkylphosphite, Tris(nonylphenyl)-phosphit, Trilaurylphosphit, Trioctadecylphosphit, Distearyl-pentaerythritdiphosphit, Tris(2,4-di-tert-butylphenyl)-phosphit, Diisodecylpentaerythrit-diphosphit, Bis(2,4-di-tert-butylphenyl)-pentaerythritdiphosphit, Bis- (2,6-di-tert-butyl-4methylphenyl)-pentaerythritdiphosphit, Bis-isodecyloxy-pentaerythritdiphosphit, Bis(2,4-ditert-butyl-6-methylphenyl)-pentaerythritdiphosphit, Bis-(2,4,6-tri-tert-butylphenyl)-pentaerythritdiphosphit, Tristearyl-sorbit-triphosphit, Tetrakis(2,4-di-tert-butylphenyl)-4,4'-biphenylen-diphosphonit, 6-lsooctyloxy-2,4,8,10-tetra-tert-butyl-12H-dibenz[d,g]-1,3,2-dioxaphosphocin, 6-Fluor-2,4,8,10-tetra-tert-butyl-12-methyl-dibenz[d,g]-1,3,2-dioxaphosphocin, Bis(2,4-di-tert-butyl-6-methylphenyl)-methylphosphit, Bis(2,4-di-tert-butyl-6-methylphenyl)-ethylphosphit.
5. Hydroxylamine wie z.B. N,N-Dibenzylhydroxylamin, N,N-diethylhydroxylamin, N,N-Dioctylhydroxylamin, N,N-Dilaurylhydroxylamin, N,N-Ditetradecylhydroxylamin, N,N-Dihexadecylhydroxylamin, N,N-Dioctadecylhydroxylamin, N-Hexadecyl-N-octadecylhydroxylamin, NHeptadecyl-N-octadecylhydroxylamin, N,N-Dialkylhydroxylamin aus hydrierten Talgfettaminen.
6. Nitrone wie z.B. N-Benzyl-alpha-phenyl-nitron, N-Ethyl-alpha-methyl-nitron, N-Octyl-alpha-heptyl-nitron, N-Lauryl-alpha-undecyl-nitron, N-Tetradecyl-alpha-tridecyl-nitron, NHexadecyl-alpha-pentadecyl-nitron, N-Octadecyl-alpha-heptadecyl-nitron, N-Hexadecylalpha-heptadecyl-nitron, N-Ocatadecyl-alpha-pentadecyl-nitron, N-Heptadecyl-alphaheptadecyl-nitron, N-Octadecyl-alpha-hexadecyl-nitron, Nitrone abgeleitet von N,N-Dialkylhydroxylaminen hergestellt aus hydrierten Talgfettaminen.
7. Thiosynergisten wie z.B. Thiodipropionsäure-di-laurylester oder Thiodipropionsäure-distearylester.
8. Peroxidzerstörende Verbindungen, wie z.B. Ester der β-Thio-dipropionsäure, beispielsweise der Lauryl-, Stearyl-, Myristyl- oder Tridecylester, Mercaptobenzimidazol, das Zinksalz des 2-Mercaptobenzimidazols, Zink-dibutyl-dithiocarbamat, Dioctadecyldisulfid, Pentaerythrit-tetrakis(β-dodecylmercapto)-propionat.
9. Polyamidstabilisatoren, wie z.B. Kupfersalze in Kombination mit Jodiden und/oder Phosphorverbindungen und Salze des zweiwertigen Mangans.
10. Basische Co-Stabilisatoren, wie z.B. Melamin, Polyvinylpyrrolidon, Dicyandiamid, Triallylcyanurat, Harnstoff-Derivate, Hydrazin-Derivate, Amine, Polyamide, Polyurethane, Alkali- und Erdalkalisalze höherer Fettsäuren, beispielsweise Ca-Stearat, Zn-Stearat, Mg-Behenat, Mg-Stearat, Na-Ricinoleat, K-Palmitat, Antimonbrenzcatechinat oder Zinnbrenzcatechinat.
11. Nukleierungsmittel, wie z.B. anorganische Stoffe wie z.B. Talk, Metalloxide wie Titandioxid oder Magnesiumoxid, Phosphate, Carbonate oder Sulfate von vorzugsweise Erdalkalimetallen; organische Verbindungen wie Mono- oder Polycarbonsäuren sowie ihre Salze wie z.B. 4-tert-Butylbenzoesäure, Adipinsäure, Diphenylessigsäure, Natriumsuccinat oder Natriumbenzoat; polymere Verbindungen wie z.B. ionische Copolymerisate ("Ionomere").
12. Füllstoffe und Verstärkungsmittel, wie z.B. Calciumcarbonat, Silikate, Glasfasern, Glaskugeln, Asbest, Talk, Kaolin, Glimmer, Bariumsulfat, Metalloxide und -hydroxide, Ruß, Graphit, Holzmehl und Mehle oder Fasern anderer Naturprodukte, synthetische Fasern.
13. Sonstige Zusätze, wie z.B. Weichmacher, Gleitmittel, Emulgatoren, Pigmente, Rheologieadditive, Katalysatoren, Verlaufshilfsmittel, Optische Aufheller, Flammschutzmittel, Antistatika, Treibmittel.
14. Benzofuranone bzw. Indolinone, wie z.B. in US-A-4325863, US-A-4338244, US-A5175312, US-A-5216052, US-A-5252643, DE-A-4316611, DE-A-4316622, DE-A-4316876, EP-A-0589839 oder EP-A-0591102 beschrieben, oder 3-[4-(2-Acetoxyethoxy)phenyl]-5,7di-tert-butyl-benzofuran-2-on, 5,7-Di-tert-butyl-3-[4- (2-stearoyloxyethoxy) phenyl]-benzofuran-2-on, 3,3'-Bis[5,7-di-tert-butyl-3-(4-[2-hydroxyethoxy]phenyl)-benzofuran-2-on], 5,7-Ditert-butyl-3-(4-ethoxyphenyl) benzofuran-2-on, 3-(4-Acetoxy-3,5-dimethylphenyl)-5,7-di-tertbutyl-benzofuran-2-on, 3-(3,5-Dimethyl-4-pivaloyloxy-phenyl)-5,7-di-tert-butyl-benzofuran-2on.

Diese zusätzlichen Additive werden zweckmäßig in Mengen von 0,1-10, beispielsweise 0,25 Gew.-%, bezogen auf das zu stabilisierende Polymer, eingesetzt.

Die nachfolgenden Beispiele illustrieren die Erfindung weiter. Alle Angaben in Teilen oder Prozenten, in den Beispielen ebenso wie in der übrigen Beschreibung sowie in den Patentansprüchen, beziehen sich auf das Gewicht, sofern nichts anderes angegeben ist. In den Beispielen werden die folgenden Abkürzungen verwendet:
- CDCl₃:: Deuterochloroform
- Da:: Dalton
- GC:: Gaschromatographie
- ¹H-NMR:: Magnetische Kernresonanz des Nuklids ¹H
- MALDI - MS:: Matrix Assisted Laser Desorption/lonization - Massenspektroskopie
- Mₙ:: Zahlenmittel der Molmasse (Einheit g/Mol)
- M_{w}:: Massenmittel der Molmasse (Einheit g/Mol)
- ^{T}g^{:}: Glasübergangstemperatur
- TGA:: Thermogravimetrische Analyse - 10 %-iger Gewichtsverlust bei einer definierten Temperatur

### Herstellungsbeispiele

### B1) 4-[4-(2,3-Epoxypropoxy)phenyl]-2,2,6,6-tetramethylpiperidin

### B1a) 4-(4-Hydroxyphenyl)-2,2,6,6-tetramethyl-cyclo-1-aza-hex-3-en-hydrochlorid

In einem 750 ml Sulfierkolben werden 77,6 g (0,5 Mol) Triacetonamin und 49,8 g (0,53 Mol) Phenol bei 60°C aufgeschmolzen. In 30 Minuten werden 135 ml konzentrierte Salzsäure dazugetropft. Die gelbe Lösung wird bei 70°C 24 Stunden gerührt und dann auf Raumtemperatur abgekühlt.
Der Feststoff wird abgetrennt und mit Methylenchlorid gewaschen.
Die Kristalle werden bei 80°C im Hochvakuum getrocknet.

Ausbeute: 107 g (79 %)

Mikroanalyse: N-Gehalt 4,5 % (Theorie 5,2 %)

### B1b) 4-(4-Hydroxyphenyl)-2,2,6,6-tetramethylpiperidin

107 g (0,4 Mol) des unter B1a) hergestellten Hydrochlorids werden in 1,2 Liter Methanol gelöst, mit 13 g 5 % Platin/C-Katalysator versetzt und bei Normaldruck mit Wasserstoff reduziert. Nach 3 Stunden ist die theoretische Menge Wasserstoff (10 Liter) aufgenommen.
Man trennt den Katalysator ab. Das Lösungsmittel wird am Rotationsverdampfer abdestilliert. Der Rückstand wird in 400 ml Wasser aufgenommen und gekocht. Die feine Suspension giesst man auf 138 g (1 Mol) Kaliumcarbonat, das in 150 ml Wasser gelöst ist. Die Suspension wird dann auf Raumtemperatur abgekühlt und der Feststoff abgetrennt. Der Feststoff wird gut mit Wasser gewaschen und im Hochvakuum bei 80°C getrocknet.

Ausbeute: 75 g (80 %)

Schmelzpunkt: 211 - 213°C

Eine in Xylol umkristallisierte Probe schmilzt bei 214°C.

### B1c) 4-[4-(2,3-Epoxypropoxy)phenyl]-2,2,6,6-tetramethylpiperidin

In einem 1,5 Liter Sulfierkolben mit KPG-Rührer, Innenthermometer und Tropftrichter werden 135 g (580 mmol) der analog B1 b) hergestellten Substanz, 266 g (2,87 Mol) Epichlorhydrin und 7,8 g (23 mmol) Tetrabutylammoniumhydrogensulfat vorgelegt. Unter Rühren werden 270 g Natriumhydroxid, die in in 270 ml Wasser gelöst sind, so dazugetropft, dass die Innentemperatur bei kleiner/gleich 30°C liegt. Nach dem Zutropfen der Natronlauge lässt man noch 3 Stunden lang stark rühren. Es wird mit 200 ml Toluol verdünnt, die wässrige Phase abgetrennt und die organische Phase mit Eiswasser gewaschen. Die organische Phase wird dann mit Natriumsulfat getrocknet und anschliessend eingedampft. Der Rückstand wird über eine Kurzwegdestillationsapparatur destilliert.

Siedepunkt der Flüssigkeit: 135°C / 0,07 Torr

Ausbeute: 59 g (35 %)

Schmelzpunkt: 40°C

| Mikroanalyse | | |
|---|---|---|
| | berechnet | gefunden |
| C | 74,70 % | 74,16 % |
| H | 9,40 % | 9,73 % |
| N | 4,84 % | 5,03 % |

| ¹H-NMR (CDCl₃): | | |
|---|---|---|
| 0,6 - 0,8 ppm | (1H , s) | NH |
| 1,14 ppm und 1,27 ppm | (12H , s) | CH₃ |
| 1,18 - 1,26 ppm | (2H , m) | CH₂ ax. (Piperidin) |
| 1,71 - 1,76 ppm | (2H , m) | CH₂ eq. (Piperidin) |
| 2,72 - 2,75 ppm und | | |
| 2,87 - 2,90 ppm | (2H , m) | CH₂ (Epoxid) |
| 2,92 - 3,01 ppm | (1H , m) | CH (Piperidin) |
| 3,32 - 3,36 ppm | (1H , m) | CH (Epoxid) |
| 3,91 - 3,96 ppm und | | |
| 4,16 - 4,21 ppm | (2H , m) | -O-CH₂-Epoxid |
| 6,85 ppm und 6,88 ppm | | |
| 7,12 ppm und 7,15 ppm | (4H , d) | Aromat |

### B2) 4-[4-(2,3-Epoxypropoxy) phenyl]-1,2,2,6,6-pentamethylpiperidin

### B2a) 4-[4-Trimethylsilyloxy]phenyl-2,2,6,6-tetramethylpiperidin

In einem 1,5 Liter Sulfierkolben mit KPG-Rührer, Innenthermometer und Tropftrichter werden unter Argon 70 g (0,3 Mol) der analog B1 b) hergestellten Substanz in 700 ml Toluol gelöst. Dazu gibt man 105 ml Triethylamin (0,75 Mol) und erwärmt auf 80°C. Dann tropft man langsam 35,8 g (0,33 Mol) Trimethylchlorsilan dazu und lässt bei 80°C noch 20 Stunden rühren. Es wird abgekühlt und vom unlöslichen Hydrochloridsalz abgetrennt. Man dampft vom Lösungsmittel ab und destilliert den Rückstand im Hochvakuum. Man erhält 38 g (42 %) einer Flüssigkeit mit Siedepunkt 120°C / 0,01 Torr.

| Mikroanalyse : | | |
|---|---|---|
| | berechnet | gefunden |
| C | 70,76 % | 70,71 % |
| H | 10,23 % | 10,54 % |
| N | 4,58 % | 4,56 % |
| Si | 9,19% | 9,20 % |

### B2b) 4-(4-Hydroxyphenyl)-1,2,2,6,6-pentamethylpiperidin

In einem 250 ml Rundkolben werden unter Stickstoff 30,6 g (0,1 Mol) der unter B2a) hergestellten Substanz, 13,8 g (0,11 Mol) Dimethylsulfat und 17,9 g (0,13 Mol) Kaliumcarbonat in 150 ml Methylethylketon gelöst und 20 Stunden am Rückfluss gekocht. Es wird zuerst vom Feststoff abgetrennt und dann vom Lösungsmittel befreit. Das verbleibende Oel wird in 100 ml Essigsäure gelöst und in konzentrierter Kaliumcarbonatlösung gefällt. Der Niederschlag wird abgetrennt und mit Wasser gewaschen. Die trockene Substanz wird in n-Hexan umkristallisiert.

Ausbeute: 20,2 g (82 %)

Schmelzpunkt: 145°C

| Mikroanalyse : | | |
|---|---|---|
| | berechnet | gefunden |
| C | 77,68 % | 77,71 % |
| H | 10,49 % | 10,33 % |
| N | 5,66% | 5,83 % |

### B2c) 4-[4-(2,3-Epoxypropoxy) phenyl]-1,2,2,6,6-pentamethylpiperidin

In einem 250 ml Rundkolben, mit Magnetrührer und Kühler versehen, werden 18,6 g (75 mmol) der unter B2b) hergestellten Substanz in 50 ml Epichlorhydrin während 4 Stunden bei 70°C gerührt. Danach wird überschüssiges Epichlorhydrin entfernt und das gelbe Oel in wenig Wasser emulgiert und mit 30 ml 50%iger Natronlauge bei 70°C gerührt. Die abgekühlte Lösung wird mit Methylenchlorid extrahiert. Die organische Phase wird getrocknet und anschliessend eingedampft. Der Rückstand wird im Kugelrohr destilliert.

Man erhält 9,5 g (42 %) eines hellgelben Oels mit Siedepunkt 150°C / 0,01 Torr.

| Mikroanalyse : | | |
|---|---|---|
| | berechnet | gefunden |
| C | 75,21 % | 75,17% |
| H | 9,63 % | 9,57 % |
| N | 4,62 % | 4,69 % |

Analog den obigen Herstellungsbeispielen B1a), B1b) und B2a) und B2b) werden folgende Substanzen hergestellt **[Tabelle 1]**:

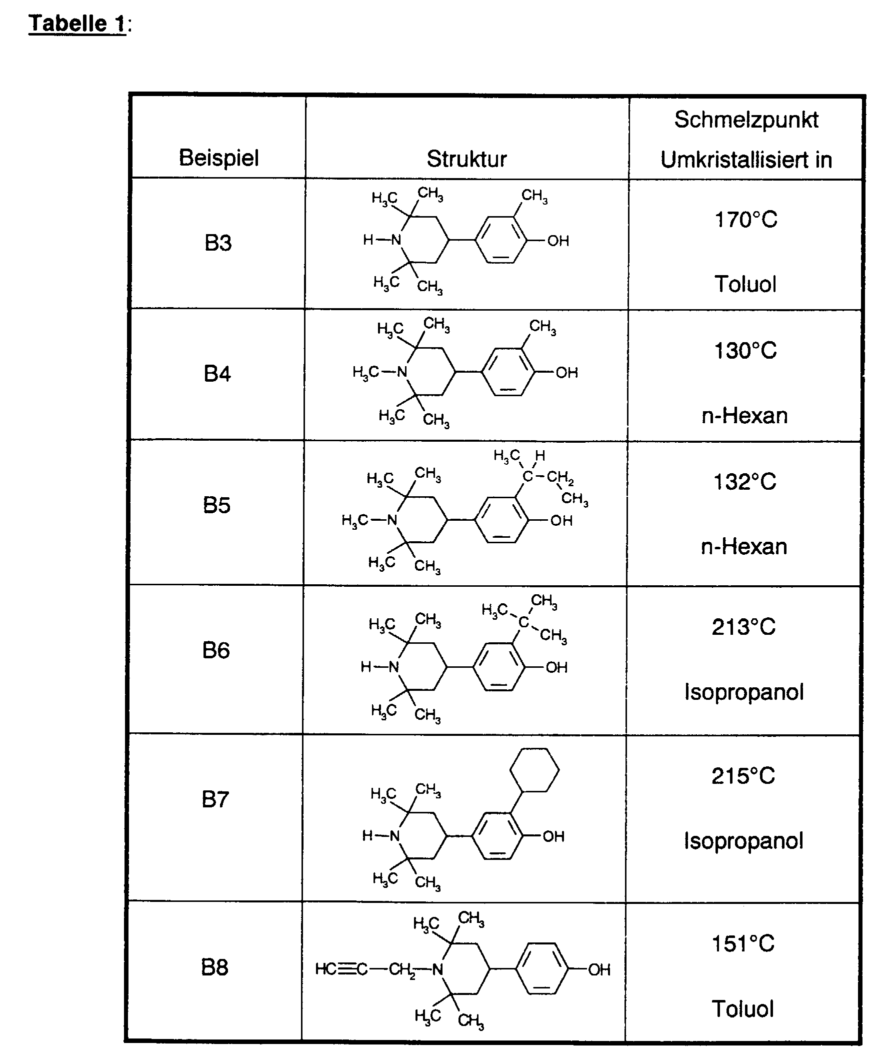

### B9) 4-[4-(2,3-Epoxypropoxy)phenyl]-1-propargyl-2,2,6,6-tetramethylpiperidin

In einem 750 ml Sulfierkolben, mit KPG-Rührer und Innenthermometer versehen, werden 64 g (236 mmol) der Verbindung B8, 87,3 g (943 mmol) Epichlorhydrin, 300 ml 50%ige Natronlauge, 200 ml Methylenchlorid und 3,2 g (9,4 mmol) Tetrabutylammoniumhydrogensulfat vorgelegt. Diese Mischung wird bei Raumtemperatur stark gerührt. Nach 4 Stunden wird die Reaktionsmischung über eine Glasfilternutsche filtriert und die organische Phase abgetrennt. Nach dem Abdampfen der Lösungsmittel wird der Rückstand in n-Hexan heiss gelöst und umkristallisiert.

Man erhält 22 g (28 %) eines Festkörpers, der bei 71°C schmilzt.

| Mikroanalyse : | | |
|---|---|---|
| | berechnet | gefunden |
| C | 77,02 % | 76,95 % |
| H | 8,93 % | 8,97 % |
| N | 4,28 % | 4,37 % |

| ¹H-NMR (CDCl₃): | | |
|---|---|---|
| 1,13 ppm und 1,26 ppm | (12H, s) | CH₃ |
| 1,57 - 1,70 ppm | (4H, m) | CH₂ (Piperidin) |
| 2,13 - 2,14 ppm | (1H, t) | C≡C-H |
| 2,73 - 2,76 ppm und | | |
| 2,88 - 2,92 ppm | (2H, m) | CH₂ (Epoxidring) |
| 2,85 - 2,96 ppm | (1H, m) | Ar-CH< |
| 3,32 - 3,37 ppm | (1H, m) | CH (Epoxidring) |
| 3,36 - 3,37 ppm | (2H, d) | N-CH₂ |
| 3,91 - 3,97 ppm und | | |
| 4,16 - 4,21 ppm | (2H, m) | O-CH₂- |
| 6,83 - 6,88 ppm und | | |
| 7,12 - 7,17 ppm | (4H, m) | Ar-H |

### B10) 4-[4-(2,3-Epoxypropoxy)-3-methylphenyl]- ,2,2,6,6-pentamethylpiperidin

In einem 200 ml Sulfierkolben mit Magnetrührer und Thermometer werden 20 ml Epichlorhydrin, 50 ml 50-%ige Natronlauge und 0,23 g Tetrabutylammonium-hydrogensulfat vorgelegt. Zu dieser Emulsion werden bei Raumtemperatur 4,5 g (17,2 mmol) der Substanz B4 zugegeben. Nach 6 Stunden ist der Umsatz vollständig. Die organische Phase wird abgetrennt, getrocknet und eingedampft.
Der Rückstand wird im Kugelrohr destilliert.
Man erhält 2,7 g (50 %) einer klaren Flüssigkeit mit einem Siedepunkt von ca. 200°C bei 0,01 Torr.

| Mikroanalyse : | | |
|---|---|---|
| | berechnet | gefunden |
| C | 75,67 % | 75,52 % |
| H | 9,84 % | 9,91 % |
| N | 4,41 % | 4,45 % |

| ¹H-NMR (CDCl₃): | | |
|---|---|---|
| 1,09 und 1,16 ppm | (12H, s) | CH₃ (Piperidin) |
| 1,55 - 1,67 ppm | (4H, m) | CH₂ (Piperidin) |
| 2,23 ppm | (3H, s) | CH₃ (Aromat) |
| 2,29 ppm | (3H, s) | N-CH₃ |
| 2,75 - 2,90 ppm | (3H, m) | CH₂ (Epoxid) und |
| | | C-H (Piperidin) |
| 3,32 - 3,37 ppm | (1H, m) | C-H (Epoxid) |
| 3,92 - 3,98 ppm und 4,16 - 4,21 ppm | (2H, m) | O-CH₂ |
| 6,73 - 6,76 ppm | (1H, d) | C-H (Aromat) |
| 6,98 - 7,02 ppm | (2H, m) | C-H (Aromat) |

### B10a) 4-[4-(2,3-Epoxypropoxy)-3-tert.-butyl-phenyl]-2,2,6,6-tetramethylpiperidin

Analog dem Beispiel B10 werden 4,9 g (17mmol) des Phenols B6 mit 20 ml Epichlorhydrin umgesetzt. Das Rohprodukt wird im Kugelrohr destilliert. Man erhält 2 g (34%) einer farblosen Flüssigkeit, die bei 170°C/0,01 Torr siedet. Die Substanz wird spontan fest und besitzt einen Schmelzpunkt von 88°C.

| Mikroanalyse : | | |
|---|---|---|
| | berechnet | gefunden |
| C | 76,48 % | 75,75 % |
| H | 10,21 % | 10,34 % |
| N | 4,05 % | 3,84 % |

| ¹H-NMR (CDCl₃): | | |
|---|---|---|
| 0,72 ppm | (1H, s) | NH |
| 1,15 ppm | (6H, s) | CH₃ (Piperidin) |
| 1,19 - 1,23 ppm | (2H, m) | CH₂ (Piperidin) |
| 1,28 ppm | (6H, s) | CH₃ (Piperidin) |
| 1,41 ppm | (9H, s) | tert.-Butyl |
| 1,72 - 1,77 ppm | (2H, m) | CH₂ (Piperidin) |
| 2,76 - 2,79 und 2,92 - 2,94 ppm | (2H, m) | O-CH₂- (Epoxydring) |
| 2,95 - 3,00 ppm | (1H, m) | C-H (Piperidin) |
| 3,37 - 3,42 ppm | (1H, m) | CH-O (Epoxydring) |
| 3,96 - 4,02 und 4,20 - 4,25 ppm | (2H, m) | -CH₂-O- |
| 6,77 - 6,80 und 6,99 - 7,02 | | |
| und 7,12 - 7,13 ppm | (3H, m) | C-H (Aromat) |

### B11) Poly{4-{4-(2,3-Epoxypropoxy)-3-methylphenyl]-1,2,2,6,6-pentamethylpiperidin}

In einem 10 ml Rundkolben werden 2 g (6,3 mmol) der unter B10 hergestellten Substanz mit 30 mg (0,3 mmol) Kalium-tert.-butylat und 2 g Toluol vorgelegt und während 8 Stunden bei 110°C erhitzt. Eine GC-Analyse zeigt kein Monomer mehr. Das Lösungsmittel wird abgedampft und der Rückstand im Hochvakuum getrocknet.

Man erhält 2 g (100 %) eines Feststoffs.

| Mikroanalyse : | | |
|---|---|---|
| | berechnet | gefunden |
| C | 75,67 % | 74,59 % |
| H | 9,84 % | 9,85 % |
| N | 4,41 % | 4,09 % |

TGA (Luft, 2°C/min Aufheizgeschwindigkeit): 10 % Gewichtsverlust bei 284°C

MALDI-MS: Mₙ = 4000 , M_{w} = 6100

### B12) Poly{4-[4-(2,3-Epoxypropoxy)phenyl]-2,2,6,6-tetramethylpiperidin}

In einem 250 ml Rundkolben, mit Magnetrührer und Kühler versehen, werden unter Argongas 37 g (128 mmol) der unter Beispiel B1c) hergestellten Verbindung mit 0,3 g (2,7 mmol) Kalium-tert.-butanolat und 80 ml Toluol vorgelegt. Man lässt bei Rückfluss während 8 Stunden reagieren und kühlt anschliessend auf Raumtemperatur ab. Das Polymer wird durch Eintragen der Reaktionslösung in 700 ml Acetonitril gefällt. Das Polymer wird anschliessend bei 100°C während 3 Stunden im Hochvakuum getrocknet.

Ausbeute: 33 g (89 %)

Maximum des Schmelzbereichs im DSC: 63°C (DSC, N₂, 10°C/Min.)

| Mikroanalyse : | | |
|---|---|---|
| | berechnet | gefunden |
| C | 74,70 % | 73,75 % |
| H | 9,40 % | 9,18 % |
| N | 4,84 % | 4,91 % |

TGA (Luft, 2°C/Min.): 10 % Gewichtsverlust bei 335°C

MALDI-MS: Mn = 3500 , Mw = 6000

### B13) Terpolymer A

In einem 100 ml Rundkolben, der mit einem Magnetrührer, einem Rückflusskühler und einem Tropftrichter ausgestattet ist, werden unter Argon 7 g (24 mmol) der unter B1 c) hergestellten Verbindung und 330 mg (2,9 mmol) Kalium-tert.-butylat in 10 ml Toluol vorgelegt. Die Reaktionsmischung wird am Rückfluss 5 Stunden lang gerührt. Eine Gaschromatographie-Analyse zeigte den vollständigen Umsatz der Verbindung B1 c). Ueber einen Tropftrichter werden nun 5,5 g (24 mmol) 4-(2,3-Epoxypropoxy)-1,2,2,6,6-pentamethylpiperidin und 5,2 g (24 mmol) 4-(2,3-Epoxypropoxy)- 2,2,6,6-tetramethylpiperidin in 8 ml Toluol zur heissen Reaktionslösung gegeben und lässt bei Rückfluss weitere 17 Stunden lang rühren. Das GC zeigt kein Monomer mehr an.
Die Reaktionslösung wird abgekühlt und das Toluol vollständig abgedampft. Man erhält 18 g (100 %) eines halbfesten Polymers mit einem Glasübergangspunkt (T_{g}) von 21°C.

Im TGA (2°C/Min., Luft) ist ein 10%-iger Gewichtsverlust bei 298°C festzustellen.

MALDI-MS: Mₙ = 2190 , M_{w} = 2930

### B14) Terpolymer B

Wie unter B13) beschrieben, werden dieselben Mengen Monomere, Lösungsmittel und Initiator gleichzeitig vorgelegt und polymerisiert. Nach 6 Stunden zeigt das GC keine Monomeren mehr an.
Man erhält nach dem Abdampfen des Lösungsmittels 18 g (100 %) eines Polymeren, das einen Glasübergangspunkt (T_{g}) von 21,5°C aufweist.

Das TGA (2°C/Min., Luft) zeigt einen 10%-igen Gewichtsverlust bei 302°C.

MALDI-MS: Mₙ = 4800 , M_{w} = 6190

### B15) Addukt aus 4-[4-(2,3-Epoxypropoxy)phenyl]-1-propargyl2,2,6,6-tetramethylpiperidin und 4,4'-Diaminodiphenylsulfon

In einem 100 ml Rundkolben mit Magnetrührer werden unter Argon 10,7 g (43 mmol) 4,4'-Diaminodiphenylsulfon und 45,2 g (180 mmol) der unter B9) hergestellten Substanz vorgelegt. Die Mischung wird 24 Stunden lang auf 180°C erhitzt. Anschliessend wird aus der Reaktionsmischung überschüssiges
4-[4-(2,3-Epoxypropoxy)phenyl]-1-propargyl-2,2,6,6-tetramethylpiperidin im Hochvakuum abgetrennt. Der abgekühlte Rückstand wird pulverisiert.

Ausbeute: 44,2 g (82 %)

Schmelzpunkt: 61°C

| Mikroanalyse : | | |
|---|---|---|
| | berechnet | gefunden |
| C | 68,97 % | 67,91 % |
| H | 9,00 % | 8,83 % |
| N | 6,70 % | 6,44 % |
| S | 2,56 % | 2,52 % |

TGA (Luft, 2°C/Min. Aufheizgeschwindigkeit): 10 % Gewichtsverlust bei 300°C

MALDI-MS: Intensivstes lonensignal bei 1254,8 Da

### Beispiel I: Lichtstabilisierung von Polypropylenfasern

2,5 g des erfindungsgemäßen Stabilisators aus Beispiel B12) werden zusammen mit 1 g Tris(2,4-di-tert.butylphenyl)phosphit, 1 g Calcium-Monoethyl-3,5-di-tert.butyl-4-hydroxybenzylphosphonat, 1 g Calciumstearat und 2,5 g TiO₂ (Kronos RN 57) in einem Turbomischer mit 1000 g Polypropylenpulver vermischt (Schmelzindex 12 g/10 min, gemessen bei 230°C/2,16 kg). Die Mischungen werden bei 200-230°C zu Granulat extrudiert; dieses wird anschließend mit Hilfe einer Pilotanlage (Leonard; Sumirago/VA, Italy) unter folgenden Bedingungen zu Fasern verarbeitet:

| | |
|---|---|
| Extrudertemperatur | 190-230°C |
| Kopftemperatur | 255-260°C |
| Streckverhältnis | 1 :3,5 |
| Strecktemperatur | 120°C |
| Fasern | 12 den |

Die so hergestellten Fasern werden vor weißem Hintergrund in einem Weather-O-Meter® Typ 65 WR (Atlas Corp.) mit einer Schwarztafeltemperatur von 63°C gemäß ASTM D 256585 belichtet. Nach unterschiedlichen Belichtungszeiten wird die verbliebene Zugfestigkeit der Proben gemessen. Aus den Meßwerten wird die Belichtungszeit T₅₀ berechnet, nach der die Zugfestigkeit der Proben nur noch halb so groß ist.

Zu Vergleichszwecken werden Fasern ohne erfindungsgemäßen Stablisator unter sonst gleichen Bedingungen hergestellt und getestet. Die Testergebnisse sind in Tabelle 2 zusammengestellt.

**Tabelle 2:**

| Belichtungsdauer bis zur Halbierung der anfänglichen Zugfestigkeit | |
|---|---|
| Stabilisator | Belichtungsdauer |
| kein | 300 h |
| aus Beispiel 12 | 1640 h |

Die erfindungsgemäß stabilisierten Fasern weisen einen hervorragenden Festigkeitserhalt auf.

### Beispiel II: Stabilisierung eines Zweischicht-Lackes

Der erfindungsgemässe Stabilisator aus Beispiel B1c) wird in 5-10 g Xylol eingearbeitet und in einem Klarlack folgender Zusammensetzung geprüft:

| | |
|---|---|
| Synthacryl® SC 303 ¹⁾ | 27,51 g |
| Synthacryl® SC 370 ²⁾ | 23,34 g |
| Maprenal® MF 650 ³⁾ | 27,29 g |
| Butylacetat/Butanol (37/8) | 4,33 g |
| Isobutanol | 4,87 g |
| Solvesso® 150 ⁴⁾ | 2,72 g |
| Kristallöl K-30 ⁵⁾ | 8,74 g |
| Verlaufshilfsmittel Baysil® MA⁶⁾ | 1,20 g |
| | 100,00 g |

| | |
|---|---|
| 1) Acrylatharz, Fa. Hoechst AG; 65% Lösung in Xylol/Butanol 26:9 | |
| 2) Acrylatharz, Fa. Hoechst AG; 75% Lösung in Solvesso® 100⁴⁾ | |
| 3) Melaminharz, Fa. Hoechst AG; 55% Lösung in Isobutanol | |
| 4) Hersteller: Fa. ESSO | |
| 5) Hersteller: Fa. Shell | |
| 6) 1% in Solvesso® 150; Hersteller: Fa. Bayer AG | |

Dem Klarlack wird 1% Stabilisator aus Beispiel B1c) (in Xylol), bezogen auf den Feststoffgehalt des Lackes, zugesetzt. Als Vergleich dient ein Klarlack, der kein Lichtschutzmittel enthält.

Der Klarlack wird mit Solvesso® 100 auf Spritzfähigkeit verdünnt und auf ein vorbereitetes Aluminiumblech (coil coat, Füller, silbermetallic Basislack) gespritzt und bei 130°C 30 Minuten eingebrannt. Es resultiert eine Trockenfilmdicke von 40-50 mm Klarlack.

Die Proben werden dann in einem UVCON®-Bewitterungsgerät der Fa. Atlas Corp. (UVB-313 Lampen) bei einem Zyklus von 4 h UV-Bestrahlung bei 60°C und 4 h Kondensation bei 50°C bewittert.

Die Proben werden in regelmässigen Abständen auf Risse untersucht.

Die Proben mit dem erfindungsgemäßen Stabilisator weisen eine hohe Beständigkeit gegenüber Rissbildung auf.

### Beispiel III: Stabilisierung eines photographischen Materials

0,087 g des Gelbkupplers der Formel 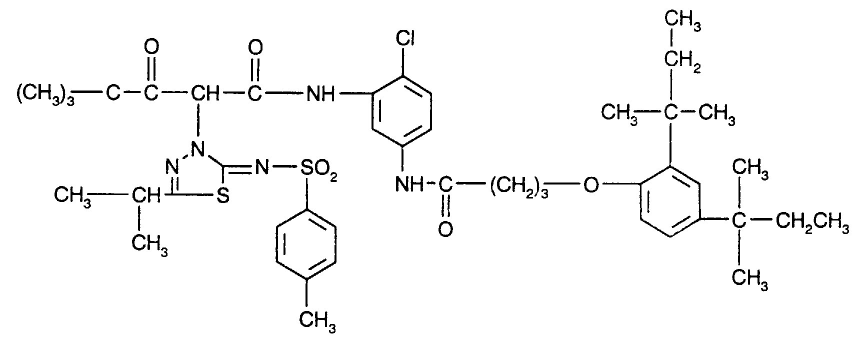 werden in 2,0 ml einer Lösung des erfindungsgemässen Stabilisators aus Beispiel B1c) in Ethylacetat (2,25 g/100 ml) gelöst. Zu 1,0 ml dieser Lösung gibt man 9,0 ml einer 2,3%-igen wässrigen Gelatinelösung, die auf einem pH-Wert von 6,5 eingestellt ist, und 1,744 g/l des Netzmittels der Formel 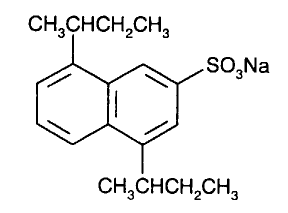 enthält.

Zu 5,0 ml der so erhaltenen Kuppleremulsion gibt man 2 ml einer Silberbromid-Emulsion mit einem Silbergehalt von 6,0 g/l sowie 1,0 ml einer 0,7%-igen wässrigen Lösung des Härters der Formel 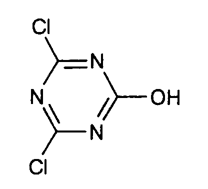 und vergiesst es auf ein 13 x 18 cm Kunststoff-beschichtetes Papier. Nach einer Härtungszeit von 7 Tagen werden die Proben hinter einem Silber-Stufenkeil mit 125 Lux·s belichtet und anschliessend im Ektaprint 2®-Prozess der Firma Kodak verarbeitet.

Die erhaltenen Gelbkeile werden in einem Atlas Weather-Ometer® mit einer 2500 WXenonlampe hinter einem UV-Filter (Kodak 2C) mit total 60 kJoule/cm² bestrahlt.

Eine Probe ohne Stabilisator läuft als Standard mit.

Der bei der Bestrahlung eingetretene Farbdichteverlust beim Absorptionsmaximum des gelben Farbstoffes wird mit einem Densitometer TR 924 A der Fa. Macbeth gemessen.

Der Lichtschutzeffekt ist aus dem Farbdichteverlust ersichtlich. Je kleiner der Dichteverlust, desto höher ist die Lichtschutzwirksamkeit.

Der erfindungsgemässe Stabilisator zeigt eine gute Lichtschutzwirkung.

### Beispiel IV: Stabilisierung von Polypropylen-Bändern

1,0 g des erfindungsgemäßen Stabilisators aus Beispiel B12) wird zusammen mit 1 g Tris(2,4-di-tert.butylphenyl)phosphit, 0,5 g Pentaerythrityl-tetrakis(3-[3',5'-di-tert.butyl-4'hydroxyphenyl]-propionat) und 1 g Calciumstearat in einem Turbomischer mit 1000 g Polypropylenpulver vermischt (STATOILMF; Schmelzindex 4,0 g/10 min, gemessen bei 230°C/2,16 kg).

Die Mischungen werden bei 200-230°C zu Granulat extrudiert; dieses wird anschließend mit Hilfe einer Pilotanlage (Leonard; Sumirago/VA, Italy) unter folgenden Bedingungen zu 2,5 mm breiten Streckbändern von 50 µm Dicke verarbeitet:

| | |
|---|---|
| Extrudertemperatur | 210-230°C |
| Kopftemperatur | 240-260°C |
| Streckverhältnis | 1 :6 |
| Strecktemperatur | 110°C |

Die so hergestellten Bänder werden vor weißem Hintergrund in einem Weather-O-Meter® Typ 65 WR (Atlas Corp.) mit einer Schwarztafeltemperatur von 63°C gemäß ASTM D 256585 belichtet. Nach unterschiedlichen Belichtungszeiten wird die verbliebene Zugfestigkeit der Proben gemessen. Aus den Meßwerten wird die Belichtungszeit T₅₀ berechnet, nach der die Zugfestigkeit der Proben nur noch halb so groß ist.

Zu Vergleichszwecken werden Bänder ohne erfindungsgemäßen Stablisator unter sonst gleichen Bedingungen hergestellt und getestet. Die Testergebnisse sind in Tabelle 3 zusammengestellt.

**Tabelle 3:**

| Belichtungsdauer bis zur Halbierung der anfänglichen Zugfestigkeit | |
|---|---|
| Stabilisator | Belichtungsdauer |
| kein | 500 h |
| aus Beispiel 12 | 1510 h |

Die erfindungsgemäß stabilisierten Bänder weisen einen hervorragenden Festigkeitserhalt auf.

## Patentansprüche

1. Verbindungen der Formel I 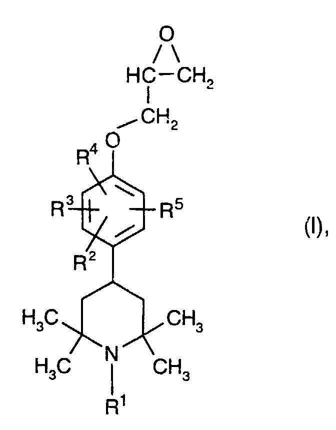 wobei
R¹ H, C₁-C₁₂-Alkyl, C₂-C₁₂-Alkenyl, C₂-C₁₂-Alkinyl, O-C₁-C₁₂-Alkyl, CO-C₁-C₁₂-Alkyl, wobei in den vorgenannten Resten Alkylketten auch durch O, S, S=O, SO₂ oder N-C₁-C₁₂-Alkyl unterbrochen sein können, C₅-C₁₂-Cycloalkyl, welches unsubstituiert oder durch 1 bis 4 C₁-C₄-Alkyl- und/oder C₁-C₄-Alkoxyreste substituiert ist, O-C₅-C₁₂-Cycloalkyl, welches unsubstituiert oder durch 1 bis 4 C₁-C₄-Alkyl- und/oder C₁-C₄-Alkoxyreste substituiert ist,
CO-C₆-C₁₄-Aryl, das unsubstituiert oder durch 1 bis 9 C₁-C₄-Alkyl- und/oder C₁-C₄-Alkoxyreste substituiert ist, O-Benzyl, das am aromatischen Kern unsubstituiert oder durch 1 bis 4 C₁-C₄-Alkyl- und/oder C₁-C₄-Alkoxyreste substituiert ist, CO-Benzyl, das am aromatischen Kern unsubstituiert oder durch 1 bis 4 C₁-C₄-Alkyl- und/oder C₁-C₄-Alkoxyreste substituiert ist, und
R², R³, R⁴ und R⁵ unabhängig voneinander H, C₁-C₁₂-Alkyl, C₅-C₁₂-Cycloalkyl, O-C₁-C₁₂-Alkyl, O-Phenyl, Halogen oder NO₂ bedeuten.

2. Verbindung gemäss Anspruch **1**, wobei
R¹ H, C₁-C₄-Alkyl, C₂-C₈-Alkenyl, C₂-C₄-Alkinyl, O-C₁-C₄-Alkyl, CO-C₁-C₄-Alkyl oder O-C₅-C₆-Cycloalkyl ist.

3. Verbindung gemäss Anspruch **1**, wobei
R¹ H, C₁-C₁₂-Alkyl, C₂-C₁₂-Alkenyl, C₂-C₁₂-Alkinyl, CO-C₁-C₁₂-Alkyl, wobei in den vorgenannten Resten Alkylketten auch durch O, S, S=O, SO₂ oder N-C₁-C₁₂-Alkyl unterbrochen sein können, und
R², R³, R⁴ und R⁵ unabhängig voneinander H, C₁-C₁₂-Alkyl oder O-C₁-C₁₂-Alkyl bedeuten.

4. Verbindung gemäss Anspruch **1**, wobei
R¹ H, CH₃, CH₂-C≡CH oder CO-CH₃
und
R², R³, R⁴ und R⁵ unabhängig voneinander H oder CH₃ bedeuten.

5. Homopolymer, Copolymer oder Terpolymere, erhältlich durch Additionspolymerisation von einer oder mehreren Verbindungen der Formel I gemäss Anspruch **1**.

6. Verbindungen, erhältlich durch Umsetzung einer oder mehrerer Verbindungen der Formel I gemäss Anspruch **1** mit mindestens einer weiteren nicht der Formel I entsprechenden Mono-Glycidylverbindung.

7. Verbindungen, erhältlich durch Umsetzung einer oder mehrerer Verbindungen der Formel I gemäss Anspruch **1** mit mindestens einem Amin oder einer Carbonsäure oder einem Phenol oder Dicarbonsäureanhydrid oder Alkoholat.

8. Verbindungen der Formel I gemäss Anspruch **1**, die an einen Füllstoff adsorbiert sind.

9. Verbindungen gemäss Anspruch **8**, wobei der Füllstoff TiO₂, SiO₂, CaCO₃, CaSO₄, BaSO₄, Al(OH)₃, Talk, Russ, Glasfasern oder -kugeln, Cellulose oder Holzmehl ist.

10. Zusammmensetzung enthaltend a) ein gegen Schädigung durch Licht, Sauerstoff und/oder Wärme empfindliches organisches Material und b) als Stabilisator mindestens eine Verbindung gemäss Anspruch **1** und/oder ein Polymer gemäss den Ansprüchen **5** bis **7**.

11. Zusammmensetzung enthaltend a) ein gegen Schädigung durch Licht, Sauerstoff und/oder Wärme empfindliches organisches Material und b) als Stabilisator mindestens einen Füllstoff gemäss Anspruch **8**.

12. Zusammensetzung nach Anspruch **10**, worin die Komponente a) eine organisches Polymer ist.

13. Zusammensetzung nach Anspruch **10**, worin die Komponente a) ein synthetisches Polymer ist.

14. Zusammensetzung nach Anspruch **10**, worin die Komponente a) ein Polyolefin oder ein Lackbindemittel auf der Basis von Acryl-, Alkyd-, Polyurethan-, Polyester- oder Polyamidharz oder entsprechend modifizierter Harze ist.

15. Zusammensetzung nach Anspruch **10** enthaltend zusätzlich zu den Komponenten a) und b) weitere übliche Additive.

16. Zusammensetzung nach Anspruch **10** enthaltend 0,01 bis 10 Gew.-% der Komponente b) bezogen auf das Gewicht der Zusammensetzung.

17. Zusammensetzung nach Anspruch **11**, worin die Komponente a) eine organisches Polymer ist.

18. Zusammensetzung nach Anspruch **11**, worin die Komponente a) ein synthetisches Polymer ist.

19. Zusammensetzung nach Anspruch **11**, worin die Komponente a) ein Polyolefin oder ein Lackbindemittel auf der Basis von Acryl-, Alkyd-, Polyurethan-, Polyester- oder Polyamidharz oder entsprechend modifizierter Harze ist.

20. Zusammensetzung nach Anspruch **11** enthaltend zusätzlich zu den Komponenten a) und b) weitere übliche Additive.

21. Zusammensetzung nach Anspruch **11** enthaltend 0,01 bis 10 Gew.-% der Komponente b) bezogen auf das Gewicht der Zusammensetzung.

22. Verfahren zur Stabilisierung von organischem Material gegen Schädigung durch Licht, Sauerstoff und/oder Wärme, dadurch gekennzeichnet, dass man diesem Material eine Verbindung gemäss Anspruch **1** und/oder ein Polymer gemäss Anspruch **5** beimischt.

23. Verfahren zur Stabilisierung von organischem Material gegen Schädigung durch Licht, Sauerstoff und/oder Wärme, dadurch gekennzeichnet, dass man diesem Material eine Verbindung gemäss Anspruch **1** und/oder ein Polymer gemäss den Ansprüchen **6** bis **8** beimischt.

24. Verwendung von Verbindungen gemäss Anspruch **1** und/oder gemäss Anspruch **5** zur Stabilisierung von organischem Material gegen Schädigung durch Licht, Sauerstoff und/oder Wärme.

25. Verwendung von Verbindungen gemäss Anspruch **1** und/oder gemäss den Ansprüchen **6** bis **8** zur Stabilisierung von organischem Material gegen Schädigung durch Licht, Sauerstoff und/oder Wärme.
